# EUROPEAN PATENT APPLICATION

(11) **EP 4 817 597 A1**
(43) Date of publication of application: **07.10.2026**
(21) Application number: 25382323.1
(22) Date of filing: 01.04.2025
(51) Int. Cl.: A61K 9/00, A61K 47/34

(54) **CATECHOLAMINE-BASED MEMBRANE FOR USE IN THE TREATMENT OF CANCER**

(71) Applicant: Fundació Institut Català de Nanociència i Nanotecnologia (ICN2), 08193 Bellaterra (ES); Universitat Autònoma de Barcelona, 08193 Bellaterra (Cerdanyola del Vallès) (ES); Consejo Superior De Investigaciones Científicas - CSIC, 28006 Madrid (ES); Fundació Institut d'Investigació Biomèdica de Bellvitge (IDIBELL), 08908 L'Hospitalet de Llobregat (ES)
(72) Inventor: SUÁREZ GARCÍA, Salvio, 08940 CORNELLÀ DE LLOBREGAT (ES); RUIZ MOLINA, Daniel, 08206 SABADELL (ES); YUSTE MATEOS, Víctor José, 08980 SANT FELIU DE LLOBREGAT (ES); BOLAÑOS CARDET, José Daniel, 08904 L'HOSPITALET DE LLOBREGAT (ES); BRUNA ESCUER, Jordi, 08011 BARCELONA (ES)
(74) Representative: ZBM Patents - Zea, Barlocci & Markvardsen

(57) **Abstract**

The present invention provides a catecholamine-based membrane for use in the treatment of cancer, wherein the self-standing catecholamine-based membrane is obtainable by a process comprising the steps of: (a) cross-linking a catechol derivative with an amine selected from the group consisting of: a known aliphatic amine hydrocarbon of formula (II); and an aromatic amine of formula (IIbis), in a liquid medium, wherein both the catechol and the amine are soluble, at a pH comprised from 6.5 to 10, and under appropriate agitation to create a catecholamine membrane in the air/liquid interface in the absence of any support; and b) isolating the membrane resulting from step (a) from the air/liquid interface.

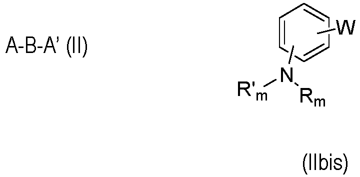

## Description

### Technical Field

The present invention relates to the field of catecholamine-based membranes. In particular, the present invention provides self-standing catecholamine-based membranes for use in the treatment of cancer.

### Background Art

Cancer remains a leading cause of mortality worldwide, and the search for effective treatments is a high priority in medical research. While conventional therapies such as chemotherapy, radiotherapy, and surgery continue to play significant roles, the need for alternative cancer therapies has grown. These alternatives aim to improve therapeutic outcomes, reduce adverse side effects, and provide options for patients with therapy-resistant cancers.

Namely, glioblastoma is the most common type of cancer that originates in the brain and has a very poor prognosis for survival. This type of cancer is characterised by its rapid growth and infiltration into surrounding brain tissue. The standard of care for glioblastoma involves a multimodality approach, combining surgery, radiation therapy, and chemotherapy. Surgical resection is often performed to remove as much of the tumour as possible, followed by radiation therapy and concurrent and adjuvant temozolomide (TMZ) chemotherapy. However, even with this aggressive treatment regimen, recurrence is the norm, generally within one year of initial therapy. The hallmark feature of glioblastoma is its infiltrative growth pattern, which makes complete surgical resection challenging and often leads to recurrence. Notably, even after seemingly complete resection of the tumour, recurrence occurs in the surgical bed. This observation has significant implications for the management and research of glioblastoma, suggesting that the surgical site, despite meticulous efforts to remove the tumour completely, retains a considerable risk of harbouring residual tumour cells.

Most of cancer treatments rely on the systemic administration of chemotherapeutics, which face obstacles in terms of effectiveness, difficulty in crossing the blood-brain barrier, and side effects, among others. Functionalized solid supports represent an innovative approach in cancer treatment, being materials modified to interact specifically with cancer cells or to deliver therapeutic agents. These materials are designed to enhance drug efficacy by improving targeting precision, reducing toxicity to surrounding tissues, and increasing the therapeutic load delivered directly to cancer cells. Examples include nanoparticles, microparticles, and nanofibers functionalized with targeting ligands or loaded with anticancer drugs. Such functionalized supports can be administered locally, releasing therapeutic agents directly at the tumour site, potentially limiting systemic toxicity and enhancing efficacy against solid tumours.

Functionalized supports, while innovative, present several challenges and their potential application in medicine may be hindered by a variety of factors. For instance, their design and manufacturing are complex and costly due to the precise modifications required, such as incorporating targeting ligands or loading drugs. Biodegradability and biocompatibility are critical to prevent long-term accumulation, reduce toxicity, and avoid adverse immune responses, while physical properties such as flexibility and adhesion are essential for effective delivery and retention at the biological tissues target sites. Additionally, ensuring ease of fabrication and reproducibility is vital for scalable manufacturing and consistent therapeutic outcomes, making the integration of these factors a complex but essential goal in biomedical engineering. Thus, there is a pressing need to develop new, more effective therapies that can target cancer cells and prevent recurrence of the tumour.

### Summary of Invention

The inventors have found that self-standing catecholamine-based membranes disclosed in PCT application WO2022258780A1 are useful in the treatment of cancer. As shown in the examples below, it has been surprisingly found that the catecholamine-based membranes according to the invention have a remarked anti-tumoral activity against different cancer cell types. Particularly, the membranes according to the invention have been proven to be highly active against glioblastoma cancer cells.

The membranes according to the invention are characterized by their excellent conformability and bioadhesion, which are features of great importance for post-surgical treatment applications. These catecholamine-based membranes can be easily inserted and removed from a surgical site without structural compromise, while conforming to the tissue surface and maintaining position in high humidity conditions. Therefore, they can be placed in the surgical bed after tumour resection to actively target tumour remanent cells and avoid recurrence, thus counteracting the infiltrative growth pattern of certain tumours.

A notable advantage of these membranes is their intrinsic antimicrobial activity, which is particularly beneficial in post-surgical settings. By reducing the risk of post-operative infections, these membranes are able to enhance patient outcomes and minimize the need for additional antibiotic treatments, thereby supporting a more successful recovery process.

As mentioned above, the catecholamine membranes of this invention exhibit cytotoxic effects against different types of cancer cells. Particularly, a catechin/hexamethylenediamine-based membrane (CATH-M) has been shown to induce significant cell death across multiple glioblastoma-derived and other cancer cell lines, demonstrating a broad cytotoxic spectrum and a remarkable anti-tumour activity.

The experiments carried out not only revealed that CATH-M exhibited superior cytotoxicity compared to temozolomide (TMZ) but also that a synergistic effect was observed when used in combination with it, suggesting that its integration into current cancer treatment protocols could improve therapeutic outcomes without disrupting existing standards of care.

Additionally, physicochemical characterization revealed that the membrane's hydroxyl and quinone groups might be responsible for the strong adhesion of the membranes which enhances its functionality. Additionally, the membranes have also been proven to be stable and have controlled biodegradability, ensuring functionality during the treatment period and reducing the risk of long-term complications.

These membranes have also shown a chemoattractant effect. This can be used as a cell trap, which further increases their therapeutic potential. This property, combined with its superior cytotoxicity, stability, and biodegradability, positions these membranes as a versatile tool in cancer treatment, whether used as an independent chemical entity or in combination with other therapies.

Thus, in an aspect, the invention provides a self-standing catecholamine-based membrane for use in the treatment of cancer, wherein the self-standing catecholamine-based membrane is obtainable by a process comprising the steps of:
a) crosslinking a catechol derivative with an amine selected from the group consisting of:
   a.1) a known aliphatic amine of formula (II)

      A-B-A' (II)

      wherein
      A and A' are the same or different and represent -NR₁R'₁,
      B represents a (C₁-C₂₀)alkylene; (C₁-C₂₀)alkylene substituted with one or more substituents selected from the group consisting of: -OH, halogen, -NO₂, cyano, -O-(C₁-C₁₀)alkyl, -C(O)OR₂, and -NR₃R'₃; (C₂-C₂₀)alkenylene; (C₂-C₂₀)alkenylene substituted with one or more substituents selected from the group consisting of: -OH, halogen, -NO₂, cyano, -O-(C₁-C₁₀)alkyl, -C(O)OR₄, and -NR₅R'₅; (C₂-C₂₀)alkynylene; (C₂-C₂₀)alkynylene substituted with one or more substituents selected from the group consisting of: -OH, halogen, -NO₂, cyano, -O-(C₁-C₁₀)alkyl, -C(O)OR₆, and -NR₇R'₇; a known 3- to 20-membered heteroalkylene; a known 3- to 20-membered heteroalkylene substituted with one or more substituents selected from the group consisting of: -OH, halogen, -NO₂, cyano, -O-(C₁-C₁₀)alkyl, -C(O)OR₈, and NR₉R'₉; a known 3- to 20-membered heteroalkenylene; and a known 3- to 20-membered heteroalkenylene substituted with one or more substituents selected from the group consisting of: -OH, halogen, -NO₂, cyano, -O-(C₁-C₁₀)alkyl, -C(O)OR₁₀, and -NR₁₁R'₁₁;
      A is bound to the first atom member forming part of the B biradical hydrocarbon backbone;
      A' is bound to the last atom member forming part of the B biradical hydrocarbon backbone;
      wherein the "first" and "last" atom members are identified reading the B biradical hydrocarbon backbone from left to right or vice versa;
      R₁, R'₁, R₃, R'₃, R₅, R'₅, R₇, R'₇, R₉, R'₉, R₁₁, and R'₁₁ are the same or different and are selected from the group consisting of: -H; (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of -OH, -NO₂, cyano, -O-(C₁-C₁₀)alkyl, -C(O)OR₁₂, and -NR₁₃R'₁₃;
      R₂, R₄, R₆, R₈, R₁₀ and R₁₂ are independently selected from the group consisting of - H; (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of -OH, -NO₂, cyano, -O-(C₁-C₁₀)alkyl, -C(O)OR₁₄and -NR₁₅R'_{15;}
      R₁₃, R'₁₃, R₁₄, R₁₅ and R'₁₅ are the same or different and are selected from the group consisting of: -H; (C₁-C₁₀)alkyl; (C₁-C₁₀)haloalkyl; (C₂-C₁₀)alkenyl; and (C₂-C₁₀)alkynyl;
      a "known 3- to 20-membered heteroalkylene" means a known saturated chain consisting of from 3 to 20 members selected from the group consisting of C(Rₓ)₂, CRₓ, -N-, -NR'ₓ-, -S-, and -O-, provided that: (a) at least one of the members is -N-, -NRₓ-, -S-, or -O-; and (b) the first and the last members forming the backbone of the heteroalkylene are carbon atoms;
      a "known 3 to 20-membered heteroalkenylene" means a known unsaturated chain consisting of from 3 to 20 members selected from the group consisting of C(Rₓ)₂, -CRₓ-, -N-, -NR'ₓ-, -S-, and -O-, provided that: (a) at least one of the members is -N-, -NRₓ-, -S-, or -O-; (b) the first and the last members forming the backbone of the heteroalkylene are carbon atoms; and (c) includes one or more double bonds;
      Rₓ is independently selected from the group consisting of -H; -OH; (C₁-C₁₀)alkyl; (C₂-C₁₀)alkenyl; (C₂-C₁₀)alkynyl; (C₁-C₁₀)haloalkyl; O-(C₁-C₁₀)alkyl; -O-(C₂-C₁₀)alkenyl; -O-(C₂-C₁₀)alkynyl; nitro, -NRₓ₁Rₓ₂; (C₁-C₁₀)alkyl substituted with one or more substituents selected from the group consisting of -OH, -NO₂, cyano, -O-(C₁-C₁₀)alkyl, -C(O)OR₁₆, and -NR₁₇R'₁₇; and halogen; and
      R_{X1}, Rₓ₂, R₁₆, R₁₇, R'₁₇, and R'ₓ are independently selected from the group consisting of -H, (C₁-C₁₀)alkyl, (C₂-C₁₀)alkenyl, (C₂-C₁₀)alkynyl, and (C₁-C₁₀)haloalkyl; and
   a.2) an aromatic amine of formula (IIbis): 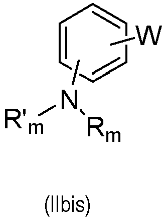
   wherein W represents:
      -NRₜR'ₜ or
      *S-S-L; wherein (*) denotes that the S atom of W radical is bound to a carbon atom forming part of the aromatic ring; and
      L is selected from the group consisting of: (C₁-C₁₀)alkyl substituted with one or more substituents selected from the group consisting of -OH, -NO₂, cyano, -O-(C₁-C₁₀)alkyl, -C(O)OR₂₈, and -NR₂₈R'₂₈, provided that at least one of the substituents is -NR₂₈R'₂₈; (C₁-C₁₀)haloalkyl substituted with one or more substituents selected from the group consisting of -OH, -NO₂, cyano, -O-(C₁-C₁₀)alkyl, -C(O)OR₂₈, and -NR₂₈R'₂₈, provided that at least one of the substituents is -NR₂₈R'₂₈; (C₂-C₁₀)alkenyl substituted with one or more substituents selected from the group consisting
      of -OH, -NO₂, cyano, -O-(C₁-C₁₀)alkyl, -C(O)OR₂₈, and -NR₂₈R'₂₈, provided that at least one of the substituents is -NR₂₈R'₂₈; (C₂-C₁₀)alkynyl substituted with one or more substituents selected from the group consisting of -OH, -NO₂, cyano, -O-(C₁-C₁₀)alkyl, -C(O)OR₂₈, and -NR₂₈R'₂₈, provided that at least one of the substituents is -NR₂₈R'₂₈; and a known aromatic ring having 5 or 6 members, each one of the members being selected from the group consisting of -CR_{y}-, -N-, -O-, -NR'_{v,} -S-;
   Rₘ, R'ₘ, Rₜ and R'ₜ are the same or different and are selected from the group consisting of: H, (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of -OH, -NO₂, cyano, -O-(C₁-C₁₀)alkyl, -C(O)OR₂₉, and -NR₃₀R'₃₀; and a known aromatic ring having 5 or 6 members, each one of the members being selected from the group consisting of -CR_{z}-, -N-, -NR'_{z}, -O-, and -S-;
   at least one of Rᵥ, is -NR₃₁R'₃₁ and the other(s) Rᵥ are selected from the group consisting of H, -NR₃₁R'₃₁, (C₁-C₁₀)alkyl; (C₁-C₁₀)haloalkyl; (C₂-C₁₀)alkenyl; (C₂-C₁₀)alkynyl; and (C₁-C₁₀)alkyl substituted with one or more substituents selected from the group consisting of -OH, -NO₂, cyano, -O-(C₁-C₁₀)alkyl, -C(O)OR₃₂, and -NR₃₃R'₃₃;
   R_{z}, and R'_{z} are independently selected from the group consisting of: H, (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of -OH, -NO₂, cyano, -O-(C₁-C₁₀)alkyl, -C(O)OR₃₄, and -NR₃₅R'₃₅; and
   R₂₈, R'₂₈, R₂₉, R₃₀, R'₃₀, R₃₁, R'₃₁, R₃₂, R₃₃, R'₃₃, R₃₄, R₃₅, and R'₃₅ are independently selected from the group consisting of: -H; (C₁-C₁₀)alkyl; (C₁-C₁₀)haloalkyl; (C₂-C₁₀)alkenyl; and (C₂-C₁₀)alkynyl;
   to create a catecholamine membrane in the air/liquid interface in the absence of any support, the crosslinking reaction being performed in a liquid medium, wherein both the catechol and the amine are soluble at a pH comprised from 6.5 to 10, particularly from 6.5 to 8 and under appropriate agitation; and
b) isolating the membrane resulting from step (a) from the air/liquid interface.

This aspect may also be formulated as a method of treatment and/or prevention of cancer, which comprises administering to a subject in need thereof, including a human, a self-standing catecholamine membrane obtainable by the process described above. It also forms part of the invention the use of a catechol and an amine as described above, for the preparation of a self-standing catecholamine membrane as described above for the treatment and/or prevention of cancer.

### Brief description of drawings

FIG. 1 shows the FTIR analysis of the synthesized membranes. FTIR spectra of A) PYRO-M, CAFF-M and GALL-M and B) DOPA-M, 4MET-M and CATH-M. Wavenumber (V) is expressed in cm⁻¹.
FIG. 2 shows the X-ray photoelectron spectroscopy curve-fitting results for C1s, N1s and O1s high-resolution XPS spectra for PYRO-M. "CPS" stands for Counts per second. Binding Energy (B.E.) is expressed in electron volts (eV).
FIG. 3 shows the X-ray photoelectron spectroscopy curve-fitting results for C1s, N1s and O1s high-resolution XPS spectra for CAFF-M. "CPS" stands for Counts per second. Binding Energy (B.E.) is expressed in electron volts (eV).
FIG. 4 shows the X-ray photoelectron spectroscopy curve-fitting results for C1s, N1s and O1s high-resolution XPS spectra for GALL-M. "CPS" stands for Counts per second. Binding Energy (B.E.) is expressed in electron volts (eV).
FIG. 5 shows the X-ray photoelectron spectroscopy curve-fitting results for C1s, N1s and O1s high-resolution XPS spectra for DOPA-M. "CPS" stands for Counts per second. Binding Energy (B.E.) is expressed in electron volts (eV).
FIG. 6 shows the X-ray photoelectron spectroscopy curve-fitting results for C1s, N1s and O1s high-resolution XPS spectra for 4MET-M. "CPS" stands for Counts per second. Binding Energy (B.E.) is expressed in electron volts (eV).
FIG. 7 shows the X-ray photoelectron spectroscopy curve-fitting results for C1s, N1s and O1s high-resolution XPS spectra for CATH-M. "CPS" stands for Counts per second. Binding Energy (B.E.) is expressed in electron volts (eV).
FIG. 8 shows the Scanning electron microscopy (SEM) images comparing the cross-section, air-face, water-face, and visual aspect inside water of PYRO-M, CAFF-M, GALL-M,DOPA-M, 4MET-M and CATH-M.
FIG. 9 shows a close view of the SEM image of CATH-M cross-section.
FIG. 10 shows SEM images of the microscopic evolution of both PYRO-M and CATH-M in different media after 8 months.
FIG. 11 shows SEM images of the microscopic evolution of CATH-M in different media after 8 months.
FIG. 12 shows SEM images of the microscopic evolution of CATH-M in pH 10 solution (Na₂CO₃/NaHCO₃).
FIG. 13 shows images of a CATH-M in an ex vivo application in a pig brain.
FIG. 14 shows a comparison of the cell death (CD) in % caused by CATH-M and temozolomide (TMZ) treatment over irradiated LN229 cells, together or separated, at different irradiation intensities (Gy) and treatment time (h).
FIG. 15 shows the cell number fold (CNF) quantification after trypsinisation of adhered LN229 cells to CATH-M or PYRO-M.
FIG. 16 shows bright field images of the LN229 cells that were detached after the cells adhered to the bottom of a treated 96-well plate (control), to a CATH-M or to a PYRO-M membrane were trypsinised.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, any ranges given include both the lower and the upper end-points of the range.

In an aspect, the present invention provides a self-standing catecholamine-based membrane for use in the treatment of cancer.

Worth to mention, through the process of the invention, the formation of the membranes takes place in the liquid-air interface, avoiding the use of substrates (e.g., glass, metals) or of the polymers which *in situ* provides the support (e.g., polyethyleneimine, PEI). The agitation contributes to the entrance of O₂ in the reaction medium, something that also determines the oxidation of the catechol derivative. And also allows for the diffusion of oxidized catechol moieties that crosslink with the amino-based ligands in the interface. The particular features of the process of the invention allow for the obtaining of a catecholamine-based membrane in the air/liquid interface. Therefore, the membrane obtainable by the process of the invention is a self-standing membrane (also so-called self-supported or free-standing membrane), which means that it does not require a support for the polymerization. In addition, the free-standing membrane of the invention shows a Janus character. They exhibited not only a different roughness depending on the side but also asymmetric chemistry. Particularly, the the side in contact with liquid medium (in the examples, water) presented higher roughness because of the creation of a nanopatterning based on catecholamine nanoparticles (produced during the process of the invention), while the air-contact side was smoother.

In the context of the present invention, the term "catecholamine-based membrane" and "catecholamine-based film" have the same meaning and can be interchangeably used and refers to a material made with a catechol derivative crosslinked with an amine compound. In the context of the invention, the catechol derivative is any compound including a moiety of formula (I), whereas the amine is an aliphatic amine of formula (II) or an aromatic amine of formula (IIbis). The crosslinking can be confirmed by any suitable routinary technique such as infrared (FT-IR) or UV-visible spectroscopy. As it is shown below, the inventors confirmed the successful cross-linking of the amine of formula (II) or (IIbis) with the catechol derivative by detecting covalent bonding formation between the catechol derivative and the amine-based ligand. This covalent interaction will depend on the functional groups of the catechol reacting with terminal amino groups. Usually, the polymerization of catechol-derivatives with amino-based molecules can be corroborated using FT-IR spectroscopy. For example, the decrease of intensity bands associated with hydroxyl groups, appearing in the range of 3600-3000 cm⁻¹, is a signal of copolymerization. Besides, the incorporation of amino-based ligands on the catechol rings forming the cross-linking can be confirmed through the appearance of new bands in the range of 1800-1650 cm⁻¹. Finally, the incorporation of aliphatic carbons present in amino-based ligands can be followed by detecting specific bands in the range of 3000-2500 cm⁻¹.

According to the present invention, the catechol derivative can be any including a moiety of formula (I). That is, the catechol derivative in the context of the invention is any compound comprising or consisting of a 6-membered aromatic ring, showing two vicinal hydroxyl groups (o-benzenediol). This catechol skeleton of formula (I) occurs in a variety of natural products such as drugs imitating them (such as MDMA), hormones/neurotransmitters, and catechin, which is found in tea. Many pyrocatechin derivatives have been suggested for therapeutic applications. Furthermore, there are many catechol derivatives commercially available from several companies (such as Sigma-Aldrich, Alfa Aesar and Fisher Scientific, among others). Illustrative non-limitative examples are pyrocatechol (CAS RN 120-80-9), pyrogallol (CAS RN 87-66-1), 4-methylcatechol (CAS RN 452-86-8), caffeic acid (CAS RN 331-39-5, 501-16-6, 71693-97-5), dopamine (CAS RN 51-61-6, 62-31-7), quercetin (CAS RN 117-39-5, 6151-25-3, 849061-97-8), hexahydroxy-triphenylene (CAS RN 4877-80-9), catechin (CAS RN 7295-85-4, 154-23-4, 18829-70-4, 225937-10-0), gallic acid (CAS RN 149-91-7, 5995-86-8), tannic acid (CAS RN 1401-55-4), and epigallocatechin gallate (CAS RN 989-51-5), among others.

In the context of the invention, the term "alkyl" (or "alkylene", when referred to "B" biradical) refers to a saturated linear or branched hydrocarbon chain containing the number of carbon atoms indicated in the claims and in the description. Examples of alkyl groups include, but are not limited to: methyl, ethyl, propyl, iso-propyl, butyl, isobutyl, tert-butyl, pentyl, hexyl, heptyl, octyl, nonanyl, decanyl, and the like.

In the context of the invention, the term "alkenyl" (or "alkenylene", when referred to "B" biradical) refers to a saturated linear or branched hydrocarbon chain containing the number of carbon atoms indicated in the claims and in the description and containing one or more double bond(s). Examples of alkenyl groups include, but are not limited to: ethenyl, propenyl, butenyl, 1-methyl-2-buten-1-yl, and the like.

In the context of the invention, the term "alkynyl" (or "alkynylene", when referred to "B" biradical) refers to a saturated linear or branched hydrocarbon chain containing the number of carbon atoms indicated in the claims and in the description, and one or more triple bond(s). Examples of alkyl groups include, but are not limited to: ethynyl, 1-propynyl, 2-butynyl, 1,3-butadinyl, 4-pentynyl, and 1-hexynyl, and the like.

The terms "known heteroalkylene" and "known heteroalkenylene" refer to heteroalkylenes and heteroalkenylenes, which are known in the art and so intend to exclude those hetero systems that are not chemically possible.

In the context of the invention, the term "known 3 to 20-membered heteroalkylene" refers to any known saturated chain comprising from 3 to 20 atoms selected from carbons and heteroatoms (i.e., atoms other than carbon atoms, such as N, O or S), provided that the first and last atoms forming the unsaturated chain are carbon atoms (to which A and A' are bound). For example, the member atoms are selected from the group consisting of -C(R_{y})₂-, -CRₓ-, -N-, -NR'ₓ-, -S-, and -O-, provided that at least one of the members is -N-, -NRₓ-, -S-, or -O-. The heteroalkylene chain can be linear or branched. Rₓ is independently selected from the group consisting of -H; -OH; (C₁-C₁₀)alkyl; (C₂-C₁₀)alkenyl; (C₂-C₁₀)alkynyl; (C₁-C₁₀)haloalkyl; O-(C₁-C₁₀)alkyl; -O-(C₂-C₁₀)alkenyl; -O-(C₂-C₁₀)alkynyl; nitro, -NRₓ₁Rₓ₂; (C₁-C₁₀)alkyl substituted with one or more substituents selected from the group consisting of -OH, -NO₂, cyano, -O-(C₁-C₁₀)alkyl, -C(O)OR₁₆, and -NR₁₇R'₁₇; and halogen. R_{X1}, Rₓ₂, R₁₆, R₁₇, R'₁₇, and R'ₓ are independently selected from the group consisting of -H, (C₁-C₁₀)alkyl, (C₂-C₁₀)alkenyl, (C₂-C₁₀)alkynyl, and (C₁-C₁₀)haloalkyl.

In the context of the invention, the term "known 3 to 20-membered "heteroalkenylene", refers to any known unsaturated chain, including one or more double bonds, and made from 3 to 20 member atoms selected from carbons and heteroatoms (i.e., atoms other than carbon atoms, such as N, O or S), provided that at least the first and last atoms forming the chain are carbon atoms (to which A and A' are bound). For example, the member atoms are selected from the group consisting of -C(R_{y})₂-, -CRₓ-, -N-, -NR'ₓ-, -S-, and -O-, provided that at least one of the members is -N-, -NR'ₓ-, -S-, or -O-. The heteroalkylene chain can be linear or branched. Rₓ is independently selected from the group consisting of -H; -OH; (C₁-C₁₀)alkyl; (C₂-C₁₀)alkenyl; (C₂-C₁₀)alkynyl; (C₁-C₁₀)haloalkyl; O-(C₁-C₁₀)alkyl; -O-(C₂-C₁₀)alkenyl; -O-(C₂-C₁₀)alkynyl; nitro, -NRₓ₁Rₓ₂; (C₁-C₁₀)alkyl substituted with one or more substituents selected from the group consisting of -OH, -NO₂, cyano, -O-(C₁-C₁₀)alkyl, -C(O)OR₁₆, and -NR₁₇R'₁₇; and halogen. R_{X1}, Rₓ₂, R₁₆, R₁₇, R'₁₇, and R'ₓ are independently selected from the group consisting of -H, (C₁-C₁₀)alkyl, (C₂-C₁₀)alkenyl, (C₂-C₁₀)alkynyl, and (C₁-C₁₀)haloalkyl.

In the context of the invention, the term "haloalkyl" refers to a saturated linear or branched hydrocarbon chain containing the number of carbon atoms indicated in the claims and in the description, wherein at least one of the carbon atoms is substituted by at least one halogen.

The number of carbon atoms of an alkyl, alkenyl, alkynyl, akylene, alkenylene, and alkynylene, is represented by a "C" (symbol of the carbon atom) with a number in subindex format, which indicates the number of carbons. Thus, "C₁" means that the alkyl has a single carbon atom; and wherein reference is made to a range, in the form, for instance, of "(C₁-C₂₀)", it means that the hydrocarbon has from 1 to 20 carbon atoms.

The term "known ring system" refers to a ring system, which is known in the art and so intends to exclude those ring systems that are not chemically possible.

According to the present invention a ring system formed by "isolated" rings means that the ring system is formed by two, three or four rings and said rings are bound via a bond from the atom of one ring to the atom of the other ring. The term "isolated" also embraces the embodiment in which the ring system has only one ring. Illustrative non-limitative examples of known ring systems consisting of one ring are those derived from: cyclopropyl, cyclobutyl, cyclopentyl, cyclohexyl, cycloheptyl, cyclopropenyl, cyclobutenyl, cyclopentenyl, phenyl, biphenylyl, and cycloheptenyl.

According to the present invention the expression "fused rings" encompasses rings totally fused, partially fused or spiro fused.

According to the present invention, when the ring system is "totally fused" it means that the ring system is formed by two, three or four rings in which two or more atoms are common to two adjoining rings. Illustrative non-limitative examples are 1,2,3,4-tetrahydronaphthyl, 1-naphthyl, 2-naphthyl, anthryl, or phenanthryl.

According to the present invention when the ring system is "partially fused", it means that the ring system is formed by three or four rings, being at least two of said rings totally fused (i.e. two or more atoms being common to the two adjoining rings) and the remaining ring(s) being bound via a bond from the atom of one ring to the atom of one of the fused rings.

According to the present invention when the ring system is "spiro fused", it means that the ring system comprises at least two rings sharing a common atom. The simplest spiro compounds are bicyclic (having just two rings), or have a bicyclic portion as part of the larger ring system, in either case with the two rings connected through the defining single common atom. Spiro compounds may be fully carbocyclic (all carbon) or heterocyclic (having one or more non-carbon atom forming part of the backbone of the rings).

In the context of the invention, the terms "halo" and "halogen" are used interchangeably and refer to a halogen group selected from the group consisting of chloro, fluoro, bromo and iodo.

In the context of the invention, the term "surgical bed" refers to the area or site in the body where the tumour was located and subsequently removed. This term is commonly used in surgery and post-operative care to describe the specific location that was operated on.

In one embodiment, the catechol derivative is one of formula (III): wherein 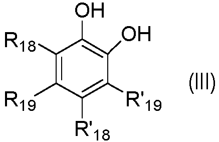
R₁₈, R₁₉, R'₁₈ and R'₁₉ are the same or different and are selected from the group consisting of: -H;
-OH;
-NR₂₀R'₂₀;
halogen
(C₁-C₁₀)alkyl;
(C₂-C₁₀)alkenyl;
(C₁-C₁₀)alkyl substituted with one or more substituents selected from the group consisting of: -OH, halogen, nitro, cyano, (C₁-C₁₀)alkyl, (C₁-C₁₀)haloalkyl, -NR₂₁R'₂₁, -C(O)OR₂₂, and -O-(C₁-C₁₀)alkyl;
(C₂-C₁₀)alkenyl substituted with one or more substituents selected from the group consisting of: -OH, halogen, nitro, cyano, (C₁-C₁₀)alkyl, (C₁-C₁₀)haloalkyl, -NR₂₃R'₂₃, -C(O)OR₂₄, and -O-(C₁-C₁₀)alkyl;
a known ring system consisting of one or two rings, each one of the rings: (a) consisting of 5 or 6 members selected from the group consisting of -C(R_{y})₂-, -CR_{y}-, -N-, -NR'_{y}, -S-, and -O-; (b) being saturated, partially unsaturated or aromatic, and (c) being isolated, partially isolated or fused;
each one of the R_{y} is independently selected from the group consisting of -H, -OH, (C₁-C₁₀)alkyl, (C₂-C₁₀)alkenyl, (C₂-C₁₀)alkynyl, (C₁-C₁₀)haloalkyl, -O-(C₁-C₁₀)alkyl, nitro, -NR₂₅R₂₅, and halogen;
R'_{y} is selected from the group consisting of -H, (C₁-C₁₀)alkyl, (C₂-C₁₀)alkenyl, (C₂-C₁₀)alkynyl, and (C₁-C₁₀)haloalkyl;
R₂₀, R'₂₀, R₂₁, R'₂₁, R₂₃, R'₂₃, R₂₅, and R'₂₅ are the same or different and are selected from the group consisting of: -H; (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of: -OH, halogen, nitro, cyano, (C₁-C₁₀)alkyl, (C₁-C₁₀)haloalkyl, -NR₂₆R'₂₆, -C(O)OR₂₇, and -O-(C₁-C₁₀)alkyl;
R₂₂ and R₂₄ are independently selected from the group consisting of: H, (C₁-C₁₀)alkyl, (C₁-C₁₀)haloalkyl, (C₂-C₁₀)alkenyl; and (C₂-C₁₀)alkynyl; and
R₂₆, R'₂₆ and R₂₇ are the same or different and are selected from the group consisting of: -H; (C₁-C₁₀)alkyl; (C₁-C₁₀)haloalkyl; (C₂-C₁₀)alkenyl; and (C₂-C₁₀)alkynyl.

In another embodiment, optionally in combination with any of the embodiments provided above or below, the catechol derivative of formula (III) is one wherein R₁₈ and R₁₉ are the same or different and are selected from the group consisting of: -H; -OH; (C₁-C₁₀)alkyl; (C₁-C₁₀)alkyl substituted as defined above; and (C₂-C₁₀)alkenyl substituted as defined above. In an alternative embodiment, optionally in combination with any of the embodiments provided above or below, the catechol derivative of formula (III) is one wherein one of R₁₈ and R₁₉ is a known ring system consisting of two rings, each one of the rings (a) consisting of 5-6 members selected from the group consisting of -C(R_{y})₂-, -CR_{y}-, -N-, -NR'_{y}-, -S-, and -O-, (b) being saturated, partially unsaturated or aromatic, and (c) being isolated, partially isolated or fused; wherein R_{y} and R'_{y} are as defined above. In an alternative embodiment, optionally in combination with any of the embodiments provided above or below, the catechol derivative of formula (III) is one wherein one of R₁₈ and R₁₉ is a known ring system consisting of two rings, each one of the rings (a) consisting of 6 members selected from the group consisting of -C(R_{y})₂-, -CR_{y}-, -N-, -NR'_{y}-, -S-, and -O-, (b) being saturated, partially unsaturated or aromatic, and (c) being fused; wherein R_{y} and R'_{y} are as defined above. In an alternative embodiment, optionally in combination with any of the embodiments provided above or below, the catechol derivative of formula (III) is one wherein one of R₁₈ and R₁₉ is a known ring system consisting of two rings, each one of the rings (a) consisting of 6 members selected from the group consisting of -C(R_{y})₂-, -CR_{y}-, -N-, -NR'_{y}-, -S-, and -O-, provided that at least one of the rings includes a heteroatom (-N-, -NR'_{y}, -S-, and -O-); (b) being saturated, partially unsaturated or aromatic, and (c) being fused; wherein R_{y} and R'_{y} are as defined above. In an alternative embodiment, optionally in combination with any of the embodiments provided above or below, the catechol derivative of formula (III) is one wherein one of R₁₈ and R₁₉ is a known ring system consisting of two rings, each one of the rings (a) consisting of 6 members selected from the group consisting of -C(R_{y})₂-, -CR_{y}-, -N-, -NR'_{y}-, -S-, and -O-, provided that at least one of the rings includes a -O- heteroatom; (b) being saturated, partially unsaturated or aromatic, and (c) being fused; wherein R_{y} and R'_{y} are as defined above. In an alternative embodiment, optionally in combination with any of the embodiments provided above or below, the catechol derivative of formula (III) is one wherein one of R₁₈ and R₁₉ is a known ring system consisting of two rings, each one of the rings (a) consisting of 6 members selected from the group consisting of -C(R_{y})₂-, -CR_{y}-, -O-, provided that at least one of the rings includes a -O-heteroatom; (b) being saturated, partially unsaturated or aromatic, and (c) being fused; wherein R_{y} and R'_{y} are as defined above. In an alternative embodiment, optionally in combination with any of the embodiments provided above or below, the catechol derivative of formula (III) is one wherein one of R₁₈ and R₁₉ is a known ring system consisting of two rings, each one of the rings (a) consisting of 6 members selected from the group consisting of -C(R_{y})₂-, -CR_{y}-, -O-, provided that only one of the rings includes a -O-heteroatom; (b) being saturated, partially unsaturated or aromatic, and (c) being fused; wherein R_{y} and R'_{y} are as defined above.

In one embodiment, optionally in combination with any of the embodiments provided above or below, the catechol of formula (III) is one of formula (Illa), (IIIb), any stereoisomer thereof, or mixtures thereof: 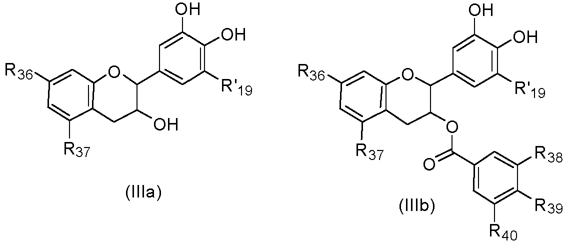 wherein
R'₁₉, R₃₆, R₃₇, R₃₈, R₃₉ and R₄₀ are the same or different and are selected from the group consisting of: -H; and -OH.

In one embodiment, optionally in combination with any of the embodiments provided above or below, the catechol of formula (III) is one of formula (Illa), wherein R₃₆ and R₃₇ are -OH, and R'₁₉ is H

In one embodiment, optionally in combination with any of the embodiments provided above or below, the catechol is selected from the group consisting of catechin, gallocatechin, epicatechin, epigallocatechin, catechin gallate, gallocatechin gallate, epicatechin gallate, epigallocatechin gallate or mixtures thereof.

In one embodiment, optionally in combination with any of the embodiments provided above or below, the catechol of formula (III) is one wherein one of the R₁₈ or R₁₉ is -H and the other is as defined in any of the above embodiments.

In one embodiment, optionally in combination with any of the embodiments provided above or below, the catechol derivative of formula (III) is one wherein R'₁₈ and R'₁₉ are -H and R₁₈ and R₁₉ are as defined in any of the above embodiments.

In one embodiment, optionally in combination with any of the embodiments provided above or below, the catechol derivative is selected from the group consisting of pyrocatechol, dopamine, pyrogallol, caffeic acid, 4-methylcatechol, and catechin.

In one embodiment, optionally in combination with any of the embodiments provided above or below, the catechol derivative is selected from the group consisting of pyrocatechol, dopamine, pyrogallol, caffeic acid and 4-methylcatechol.

In another embodiment, optionally in combination with any of the embodiments provided above or below, the amine of step a) is an aliphatic amine of formula (II).

In another embodiment, optionally in combination with any of the embodiments provided above or below, the amine is a known aliphatic amine of formula (II) wherein A and A' are the same. In another embodiment, optionally in combination with any of the embodiments provided above or below, the amine is a known aliphatic amine of formula (II) wherein A and A' are the same or different and represent NHR'₁. In another embodiment, optionally in combination with any of the embodiments provided above or below, the amine is a known aliphatic amine of formula (II) wherein A and A' are the same and represent NHR'₁. In another embodiment, optionally in combination with any of the embodiments provided above or below, the amine is a known aliphatic amine of formula (II) wherein at least one of A and A' represents -NH₂. In another embodiment, optionally in combination with any of the embodiments provided above or below, the amine is a known aliphatic amine of formula (II) wherein A and A' are the same and represent -NH₂.

In another embodiment, optionally in combination with any of the embodiments provided above or below, the amine is a known aliphatic amine of formula (II) wherein B represents (C₁-C₂₀)alkylene; (C₁-C₂₀)alkylene substituted as defined above; or a known 3- to 20-membered heteroalkylene.

In another embodiment, optionally in combination with any of the embodiments provided above or below, the amine is a known aliphatic amine of formula (II) wherein B represents (C₁-C₂₀)alkylene; (C₁-C₂₀)alkylene substituted as defined above; or a known 3- to 20-membered heteroalkylene; and A and A' are the same, such as -NH₂.

In another embodiment, optionally in combination with any of the embodiments provided above or below, the amine is a known aliphatic amine of formula (II) wherein B represents (C₁-C₁₅)alkylene; (C₁-C₁₅)alkylene substituted as defined above; or a known 3- to 15-membered heteroalkylene.

In another embodiment, optionally in combination with any of the embodiments provided above or below, the amine is a known aliphatic amine of formula (II) wherein B represents (C₁-C₁₅)alkylene; (C₁-C₁₅)alkylene substituted as defined above; or a known 3- to 15-membered heteroalkylene; and A and A' are the same, such as -NH₂.

In one embodiment, optionally in combination with any of the embodiments provided above or below, the amine is a known aliphatic amine of formula (II) wherein B represents (C₁-C₁₅)alkylene, (C₂-C₁₅)alkylene, (C₄-C₁₅)alkylene or (C₅-C₁₅)alkylene.

In one embodiment, optionally in combination with any of the embodiments provided above or below, the amine is a known aliphatic amine of formula (II) wherein B represents (C₁-C₁₅)alkylene, (C₂-C₁₅)alkylene, (C₄-C₁₅)alkylene or (C₅-C₁₅)alkylene; and A and A' are the same, such as -NH₂.

In another embodiment, optionally in combination with any of the embodiments provided above or below, the amine is a known aliphatic amine of formula (II) wherein B represents a known 3- to 15-membered heteroalkylene, particularly represents a known 3- to 10-membered heteroalkylene or a known 4- to 8-membered heteroalkylene. In another embodiment, optionally in combination with any of the embodiments provided above or below, the amine is a known aliphatic amine of formula (II) wherein B represents a known 3- to 15-membered heteroalkylene, particularly represents a 3- to 10-membered heteroalkylene; and A and A' are the same, such as -NH₂. In another embodiment, optionally in combination with any of the embodiments provided above or below, the amine is a known aliphatic amine of formula (II) wherein B represents a known 3- to 15-membered heteroalkylene, particularly represents a 3- to 10-membered heteroalkylene, wherein the members are selected from carbon, S, and N atoms, as defined above. In another embodiment, optionally in combination with any of the embodiments provided above or below, the amine is a known aliphatic amine of formula (II) wherein B represents a known 3- to 15-membered heteroalkylene, particularly represents a 3- to 10-membered heteroalkylene, wherein each one of the members is selected from the group consisting of: C(Rₓ)₂, CRₓ, -NR'ₓ-, and -S-, wherein Rₓ and R'ₓ are as defined above.

In another embodiment, optionally in combination with any of the embodiments provided above or below, the amine is a known aliphatic amine of formula (II) wherein B represents a known 3- to 15-membered heteroalkylene, particularly represents a known 3- to 10-membered heteroalkylene, wherein the members are selected from carbon, S and N atoms as defined above; and A and A' are the same, such as -NH₂.

In another embodiment, optionally in combination with any of the embodiments provided above or below, the amine is a known aliphatic amine of formula (II) wherein B represents a known 3- to 15-membered heteroalkylene, particularly represents a known 3- to 10-membered heteroalkylene, wherein two of the members are S atoms and the others are carbon atoms, as defined above. In another embodiment, optionally in combination with any of the embodiments provided above or below, the amine is a known aliphatic amine of formula (II) wherein B represents a known 3- to 15-membered heteroalkylene, particularly represents a known 3- to 10-membered heteroalkylene, wherein two of the members are S atoms and the others are carbon, as defined above; and A and A' are the same, such as -NH₂. In another embodiment, optionally in combination with any of the embodiments provided above or below, the amine is a known aliphatic amine of formula (II) wherein B represents a known 4- to 15-membered heteroalkylene, particularly represents a known 4- to 10-membered heteroalkylene, wherein the members are carbon and S atoms, as defined above. In another embodiment, optionally in combination with any of the embodiments provided above or below, the amine is a known aliphatic amine of formula (II) wherein B represents a known 4- to 15-membered heteroalkylene, particularly represents a known 4- to 10-membered heteroalkylene, wherein the members are carbon and - NR'ₓ- atoms, as defined above. In another embodiment, optionally in combination with any of the embodiments provided above or below, the amine is a known aliphatic amine of formula (II) wherein B represents a known 4- to 15-membered heteroalkylene, particularly represents a known 4- to 10-membered heteroalkylene, wherein the members are carbon and S atoms, as defined above; and A and A' are the same, such as -NH₂. In another embodiment, optionally in combination with any of the embodiments provided above or below, the amine is a known aliphatic amine of formula (II) wherein B represents a known 4- to 15-membered heteroalkylene, particularly represents a known 4- to 10-membered heteroalkylene, wherein the members are carbon and -NR'ₓ- atoms, as defined above; and A and A' are the same, such as -NH₂. In another embodiment, optionally in combination with any of the embodiments provided above or below, the amine is a known aliphatic amine of formula (II) wherein B represents a known 4- to 15-membered heteroalkylene, particularly represents a known 4- to 10-membered heteroalkylene, wherein two of the members are S atoms and the remaining members are carbon atoms, as defined above. In another embodiment, optionally in combination with any of the embodiments provided above or below, the amine is a known aliphatic amine of formula (II) wherein B represents a known 4- to 15-membered heteroalkylene, particularly represents a known 4- to 10-membered heteroalkylene, wherein one of the members is -NR'x- and the remaining members are carbon atoms, as defined above. In another embodiment, optionally in combination with any of the embodiments provided above or below, the amine is a known aliphatic amine of formula (II) wherein B represents a known 4- to 15-membered heteroalkylene, particularly represents a known 4- to 10-membered heteroalkylene, wherein two of the members are S atoms and the remaining members are carbon atoms, as defined above; and A and A' are the same, such as -NH₂. In these embodiments wherein the heteroalkylene is one including two S atoms, the heteroalkylene can also be referred as "alkylene disulfide". Illustrative non-limitative examples are cystamine, 4-aminophenyl disulfide and bis-amino polyethyleneglycol disulfide, among others.

In another embodiment, optionally in combination with any of the embodiments provided above or below, the amine is a known aliphatic amine of formula (II) wherein B represents a known 4- to 15-membered heteroalkylene, particularly represents a known 4- to 10-membered heteroalkylene, wherein one of the members is -NR'ₓ- and the remaining members are carbon atoms, as defined above; and A and A' are the same, such as -NH₂.

In another embodiment, optionally in combination with any of the embodiments provided above or below, the amine is a known aliphatic amine of formula (II), wherein B represents a known 4- to 15-membered heteroalkylene, particularly represents a known 4- to 10-membered heteroalkylene, wherein each one of the members is selected from the group consisting of C(Rₓ)₂, CRₓ, -NR'ₓ-, and -S-, wherein Rₓ and R'ₓ are as defined above.

In another embodiment, optionally in combination with any of the embodiments provided above or below, the amine is a known aliphatic amine of formula (II) wherein B represents a known 3- to 15-membered heteroalkylene, particularly represents a known 3- to 10-membered heteroalkylene, wherein:
(a) one or two of the members are -S- atoms and the others are carbon atoms selected from C(Rₓ)₂, and CRₓ; or, alternatively,
(b) one or two of the members are -NR'ₓ-, and the others are carbon atoms selected from the group consisting of C(Rₓ)₂, and CRₓ, wherein Rx is as defined above;
(c) one of the members is -S- atoms and the others are carbon atoms selected from the group consisting of C(Rₓ)₂, and CRₓ; or, alternatively,
(d) one of the members is -NR'ₓ-, and the others are carbon atoms selected from the group consisting of C(Rₓ)₂, and CR_{x;};
(e) two of the members are -S- atoms and the others are carbon atoms selected from the group consisting of C(Rₓ)₂, and CRₓ; or, alternatively,
(f) two of the members are NR'ₓ-, and the others are carbon atoms selected from the group consisting of C(Rₓ)₂, and CR_{x;};
wherein Rₓ and R'ₓ are as defined above.

In one embodiment, optionally in combination with any of the embodiments provided above or below, the known aliphatic amine of formula (II) is selected from the group consisting of an amine of formula (II1), an amino of formula (II2), and an amine of formula (II3): 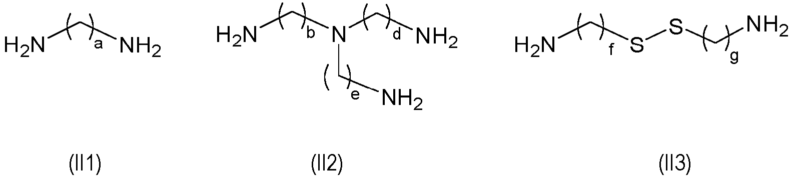 wherein a is an integer from 1 to 20; b, d, and e are independently an integer from 1 to 10, more particularly b, d, and e are the same; and f, and g are independently an integer from 1 to 10, more particularly f and g are the same.

In one embodiment, optionally in combination with any of the embodiments provided above or below, the known aliphatic amine of formula (II) is selected from the group consisting of hexamethylenediamine, octamethylenediamine, dodecamethylenediamine, cystamine, tris-(3-aminopropyl)amine, and tris-(2-aminopropyl)amine, more particularly selected from hexamethylenediamine, dodecamethylenediamine, octamethylenediamine and cystamine.

In another embodiment of the invention, optionally in combination with any of the embodiments provided above or below, the amine is an aromatic amine of formula (IIbis). In another embodiment of the invention, optionally in combination with any of the embodiments provided above or below, the amine is an aromatic amine of formula (IIbis1): 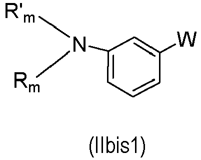 where Rₘ, R'ₘ and W are as defined above.

In another embodiment of the invention, optionally in combination with any of the embodiments provided above or below, the amine is an aromatic amine of formula (IIbis) or (IIbis1) which comprises at least two amino groups.

In another embodiment of the invention, optionally in combination with any of the embodiments provided above or below, the amine is an aromatic amine of formula (IIbis) or (IIbis1), and W represents -NRₜR'ₜ.

In another embodiment of the invention, optionally in combination with any of the embodiments provided above or below, the amine is an aromatic amine of formula (IIbis) or (IIbis1), W represents -NRₜR'ₜ, and Rₘ, R'ₘ, Rₜ and R'ₜ are the same. In another embodiment of the invention, optionally in combination with any of the embodiments provided above or below, the amine is an aromatic amine of formula (llbis) or (Ilbis1), W represents -NRₜR'ₜ, and Rₘ, R'ₘ, Rₜ and R'ₜ are the same and represent -H.

In another embodiment of the invention, optionally in combination with any of the embodiments provided above or below, the amine is an aromatic amine of formula (IIbis) or (IIbis1), W represents -NRₜR'ₜ, Rₘ and R'ₘ are the same and represent -H, and Rₜ and R'ₜ are the same and are as defined above.

In another embodiment of the invention, optionally in combination with any of the embodiments provided above or below, the amine is an aromatic amine of formula (IIbis) or (IIbis1), W represents -NRₜR'ₜ, and Rₜ and R'ₜ are the same and are other than hydrogen.

In another embodiment of the invention, optionally in combination with any of the embodiments provided above or below, the amine is an aromatic amine of formula (IIbis) or (IIbis1), W represents -NRₜR'ₜ,, and Rₘ and R'ₘ are hydrogen; and Rₜ and R'ₜ are the same and are other than hydrogen.

In another embodiment of the invention, optionally in combination with any of the embodiments provided above or below, the amine is an aromatic amine of formula (llbis) or (Ilbis1), W represents -NRₜR'ₜ, Rₘ and R'ₘ are the same and represent -H, and Rₜ and R'ₜ represent an aromatic ring as defined above under the invention. In another embodiment of the invention, optionally in combination with any of the embodiments provided above or below, the amine is an aromatic amine of formula (IIbis) or (IIbis1), W represents -NR₁R'ₜ, Rₘ and R'ₘ are the same and represent -H, and each one of Rₜ and R'ₜ represent an aromatic ring having 6 members as defined above under the invention.

In another embodiment of the invention, optionally in combination with any of the embodiments provided above or below, the amine is an aromatic amine of formula (IIbis) or (IIbis1), W represents -NRₜR'ₜ, and each one of Rₜ and R'ₜ represent an aromatic ring as defined above.

In another embodiment of the invention, optionally in combination with any of the embodiments provided above or below, the amine is an aromatic amine of formula (IIbis) or (IIbis1), W represents -NRₜR'ₜ, and each one of Rₜ and R'ₜ represent an aromatic ring having 6 members as defined above.

In another embodiment of the invention, optionally in combination with any of the embodiments provided above or below, the amine is an aromatic amine of formula (IIbis) or (IIbis1), W represents -NRₜR'ₜ, and each one of Rₜ and R'ₜ represent an aromatic ring having 6 members, all the members being -CR_{z}-, wherein R_{z} is as defined above.

In another embodiment of the invention, optionally in combination with any of the embodiments provided above or below, the amine is an aromatic amine of formula (IIbis) or (IIbis1), W represents -NRₜR'ₜ, wherein Rₜ and R'ₜ are the same and represent an aromatic ring having 6 members, all the members, being -CR_{z}-members, wherein R_{z} is as defined above.

In another embodiment of the invention, optionally in combination with any of the embodiments provided above or below, the amine is an aromatic amine of formula (IIbis) or (IIbis1), W represents -NRₜR'ₜ, Rₘ and R'ₘ are the same and represent -H, and each one of Rₜ and R'ₜ represent an aromatic ring having 6 members, all the members being -CR_{z}-, wherein R_{z} is as defined above. In another embodiment of the invention, optionally in combination with any of the embodiments provided above or below, the amine is an aromatic amine of formula (IIbis) or (IIbis1), W represents -NRₜR'ₜ, Rₘ and R'ₘ are the same and represent -H, Rₜ and R'ₜ are the same and represent an aromatic ring having 6 members, all the members, being -CR_{z}- members, wherein R_{z} is as defined above.

In another embodiment of the invention, optionally in combination with any of the embodiments provided above or below, the amine is one of formula (IIbis) or (IIbis1), being W= NRₜR'ₜ, and one or more of Rₘ, R'ₘ, Rₜ and R'ₜ having the following formula (IV): 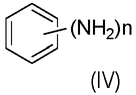 wherein n is 1 or 2, particularly 1, and the other(s) being -H.

Alternatively, in another embodiment of the invention, optionally in combination with any of the embodiments provided above or below, the amine is an aromatic amine of formula (IIbis) or (IIbis1), and W represents *S-S-L. In another embodiment of the invention, optionally in combination with any of the embodiments provided above or below, the amine is an aromatic amine of formula (IIbis) or (IIbis1), W represents *S-S-L, and Rₘ and R'ₘ are the same. In another embodiment of the invention, optionally in combination with any of the embodiments provided above or below, the amine is an aromatic amine of formula (llbis) or (Ilbis1), W represents *S-S-L, and Rₘ and R'ₘ are the same and represent -H. In another embodiment of the invention, optionally in combination with any of the embodiments provided above or below, the amine is an aromatic amine of formula (IIbis) or (IIbis1), W represents *S-S-L, Rₘ and R'ₘ are the same, and L represents an aromatic ring as defined above. In another embodiment of the invention, optionally in combination with any of the embodiments provided above or below, the amine is an aromatic amine of formula (IIbis) or (IIbis1), W represents *S-S-L, Rₘ and R'ₘ are the same and represent -H, and L represents an aromatic ring as defined above. In another embodiment of the invention, optionally in combination with any of the embodiments provided above or below, the amine is an aromatic amine of formula (llbis) or (Ilbis1), W represents *S-S-L, Rₘ and R'ₘ are the same, and L represents an aromatic ring having 6 members as defined above. In another embodiment of the invention, optionally in combination with any of the embodiments provided above or below, the amine is an aromatic amine of formula (IIbis) or (IIbis1), W represents *S-S-L, Rₘ and R'ₘ are the same and represent -H, and L represents an aromatic ring having 6 members as defined above. In another embodiment of the invention, optionally in combination with any of the embodiments provided above or below, the amine is an aromatic amine of formula (IIbis) or (IIbis1), W represents *S-S-L, Rₘ and R'ₘ are the same, and L represents an aromatic ring having 6 members, the same or different, represented by -CR_{y}-, as defined above. In another embodiment of the invention, optionally in combination with any of the embodiments provided above or below, the amine is an aromatic amine of formula (IIbis) or (IIbis1), W represents *S-S-L, Rₘ and R'ₘ are the same and represent -H, and L represents an aromatic ring having 6 members, the same or different, particularly the same, represented by -CRᵥ-, as defined above as defined above.

In another embodiment of the invention, optionally in combination with any of the embodiments provided above or below, the amine is an aromatic amine of formula (IIbis) or (IIbis1), W represents *S-S-L, and L represents an aromatic ring as defined above.

In another embodiment of the invention, optionally in combination with any of the embodiments provided above or below, the amine is an aromatic amine of formula (IIbis) or (IIbis1), W represents *S-S-L, and L represents an aromatic ring having 6 members as defined above.

In another embodiment of the invention, optionally in combination with any of the embodiments provided above or below, the amine is an aromatic amine of formula (IIbis) or (IIbis1), W represents *S-S-L, and L represents an aromatic ring having 6 members, the same or different, represented by -CR_{y}-, as defined above.

In another embodiment of the invention, optionally in combination with any of the embodiments provided above or below, the amine is an aromatic amine of formula (IIbis) or (IIbis1), W represents *S-S-L, and L has the following formula (IV): 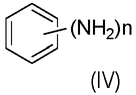 N being 1 or 2, and one of the carbon atoms forming the aromatic ring being bound to the -S- atom.

In another embodiment of the invention, optionally in combination with any of the embodiments provided above or below, the amine of formula (IIbis) is one of formula (V), (VI) or (VII):

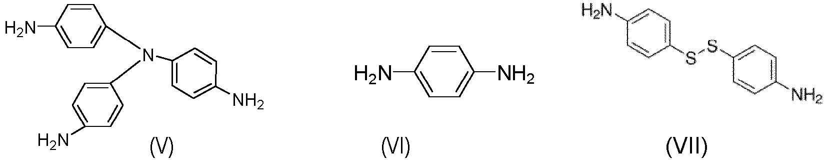

In one embodiment, optionally in combination with any of the embodiments provided above or below, the amine is selected from the group consisting of: hexamethylenediamine, octamethylenediamine, dodecamethylenediamine, cystamine, tris-(3-aminopropyl)amine, tris-(2-aminopropyl)amine, and 4,4',4"-triaminotriphenylamine.

The catechol derivative and the amine solutions can be separately prepared, and then being mixed together. Or, alternatively, one of the catechol derivative and the amine can be prepared in the form of a solution and the other one be added as directly obtained from the supplier.

The appropriate solvent system to prepare the solution(s) will depend on the polar nature of the catechol and amine, something which is part of the general knowledge of the skilled person in the art. Both, the catechol and the amine, have to dissolve in the same solvent system to carry out the crosslinking reaction. In one embodiment the catechol and amine are prepared in the form of an aqueous solution. The term "aqueous solution" embraces solutions consisting of only water but also combinations of water with other polar solvents such as water+alcohols or aqueous-based buffers. The aqueous solution is obtained by mere mixing of the compound with water. In any case, when the catechol and the amine are mixed, this step is performed under agitation (to appropriately promote the formation of the film in the air/liquid interface).

In one embodiment, optionally in combination with any of the embodiments provided above or below, the pH of the solution is comprised from 6.5 to 8 or from 7 to 7.5. In another embodiment, optionally in combination with any of the embodiments provided above or below, the pH is of 6.5, 6.6, 6.7, 6.8, 6.9, 7, 7.1, 7.2, 7.3, 7.4, 7.5, 7.6, 7.7, 7.8, 7.8, 7.9, 8, 8.1, 8.2, 8.3, 8.4, 8.5, 8.6, 8.7, 8.8., 8.9, 9, 9.1, 9.2, 9.3, 9.4, 9.5, 9.6, 9.7, 9.8, 9.9 or 10. There are well-known commercially available buffers that can be added the reaction medium to provide the intended pH value. Illustrative non-limitative examples are phosphate-buffered saline (PBS), carbonate-bicarbonate and citrate, among others. The buffer can be added in any order to the reaction medium with respect to the addition of catechol derivative and the amine.

In another embodiment of the invention, optionally in combination with any of the embodiments provided above or below, the catechol derivative is catechin and the amine is hexamethylenediamine.

In one embodiment, optionally in combination with any of the embodiments provided above or below, the liquid medium comprises an aqueous-based buffer, more particularly, an aqueous-based buffer in the absence of nitrogen atoms or amino groups, even more particularly, the aqueous-based buffer being selected from the group consisting of a phosphate buffer, a carbonate buffer and a citrate buffer.

In another embodiment, optionally in combination with any of the embodiments provided above or below, the liquid medium is water.

Once the pH was adjusted, the agitation is also adjusted. The agitation can be performed during the whole step of cross-linking. In this embodiment, as the skilled person would recognize, the speed of agitation may not provide turbulences as they would hindrance the appropriate formation of the membrane. Whether the speed is appropriate or not can be easily confirmed by the skilled person: if no turbulence is observed in the reaction medium, this will indicate that the speed is appropriate. If, when adjusted, the skilled person observed the onset of turbulences in the liquid medium, this will be indicative that the speed is not appropriate and that it should be reduced until said turbulences disappeared. Appropriate agitation means to be used during the whole cross-linking step are magnetic (or mechanical) means, using the lowest speed possible to avoid the turbulence in the air/liquid interface, being homogeneous during the whole procedure and avoiding erratic movement of the magnet or mechanical pieces involved in the agitation procedure. The skilled person can routinary determine the appropriate speed of agitation depending on the reactor's volume, liquid medium and means of agitation. For example, in case of the examples provided below, wherein the volumes were of about 20 mL, the inventors established as appropriate agitation one lower than 500 rpm using a magnetic stirrer. In one embodiment, optionally in combination with any of the embodiments provided above or below, the stirring speed is equal to or below than 450 rpm, equal to or below than 400 rpm, or equal to below than 350 rpm.

Alternatively, the agitation can be performed during part of the cross-linking reaction. In this embodiment the speed of agitation can be any: once the agitation is stopped, the reaction medium is left for a period of time such as the oxidized catechol moieties that crosslink with the amino-based ligands diffuse and arrange in the interface to form the membrane. In this alternative embodiment, the agitation can stop, for example, when a visual colour change is detected due to the oxidation of the catechol moieties. Then, it is left to complete the copolymerization reaction with the amino-based ligand, finishing with the formation of a floating membrane in the air/liquid interface.

In one embodiment, optionally in combination with any of the embodiments provided above or below, agitation is performed during the whole step of cross-linking or alternatively during part of the cross-linking reaction.

In one embodiment, optionally in combination with any of the embodiments provided above or below, agitation is performed during the whole step of cross-linking such that no turbulences are provided, more particularly at a stirring speed is equal to or below than 450 rpm, equal to or below than 400 rpm, or equal to below than 350 rpm.

In another embodiment, optionally in combination with any of the embodiments provided above or below, agitation is performed using the lowest speed possible to avoid the turbulence in the air/liquid interface, being homogeneous during the whole procedure and avoiding erratic movement of the magnet or mechanical pieces involved in the agitation procedure, more particularly at a stirring speed is equal to or below than 450 rpm, equal to or below than 400 rpm, or equal to below than 350 rpm.

In another embodiment, optionally in combination with any of the embodiments provided above or below, agitation is only performed during part of the cross-linking reaction, in particular agitation is performed such that no turbulences are provided until a change in coloration is observed and then it is stopped, even more particularly agitation is performed for a period of time from 10 min to 2 hours and then it is stopped.In another embodiment, optionally in combination with any of the embodiments provided above or below, the amine of formula (II) or (IIbis) is at a molar ratio excess with respect to the catechol derivative, particularly the molar ratio of the amine compound of formula (II) vs the catechol derivative is comprised from 1.1:1 to 3: 1, particularly from 1.2:1 to 2:1.

In another embodiment, optionally in combination with any of the embodiments provided above or below, the crosslinking step is performed at a temperature comprised from 10 to 60°C. Particularly, at a temperature from 10 to 50°C, from 12 to 40°C or from 15 to 38°C.

In another embodiment, optionally in combination with any of the embodiments provided above or below, the crosslinking step is performed for at least a period of 24 h. In one embodiment, optionally in combination with any of the embodiments provided above or below, step (a) is performed at a pH value from 7 to 7.5 and agitation equal to or below than 300 rpm. In another embodiment, optionally in combination with any of the embodiments provided above or below, step (a) is performed at a pH value from 7 to 7.5, agitation equal to or below than 300 rpm and the molar ratio of the amine compound of formula (II) vs the catechol derivative is comprised from 1.1:1 to 3:1, particularly from 1.2:1 to 2:1.

The skilled person is able to adjust routine parameters such as reaction time or concentration of reagents, which helps tuning the final thickness of the membrane.

Once the crosslinking step is finished and the catecholamine membrane has been created in the air/water or air/liquid interface in the absence of any support, it is isolated from the medium. This can be performed manually (at laboratory scale) using, for instance tweezers; or mechanically (at an industrial scale) using, for instance, ring-shaped tool.

The term "obtainable" and "obtained" have the same meaning and are used interchangeably. In any case, the expression "obtainable" encompasses the term "obtained".

The chemical asymmetry shown by the membranes of the invention provides different functionalization options and a higher versatility as platforms as already explained above. Having different chemical groups on each side of the membrane allows for the anchoring of specific molecules depending on their activity. For example, the water-contact side, which is rougher, could be functionalized with growth factors, while the air-contact side (less rougher) could be functionalized with antibacterial moieties to avoid infections during the regeneration process. For this reason, as the exposed chemical groups in each side of the membrane are different, the versatility of these membranes allows for their functionalization with (bio)molecules of different chemical nature depending on the side.

Therefore, in one embodiment, the catecholamine-based film of the invention comprises one or more molecules of interest, such as a therapeutic molecule (e.g., peptides, proteins, or antibodies, such as an antibacterial moiety (e.g., cetrimide or silver nanoparticles) or a growth factor (e.g., FGF-2 or TGFβ 3), among others) or a detection label (e.g., calcein, nanoparticles, such as metal nanoparticles or antibodies), among others.

In one embodiment, optionally in combination with any of the embodiments provided above or below, the catecholamine-based membrane further comprises one or more molecules of interest selected from the group consisting of therapeutic molecules, cells, growth factors, detection labels (fluorescent active moieties, nanoparticles or antibodies), and combinations thereof.

In one embodiment, optionally in combination with any of the embodiments provided above or below, wherein the self-standing catecholamine-based membranes for use according to the invention comprises one or more molecules of interest, the self-standing catecholamine-based membranes may be obtained by a process comprising the steps of:
(i.1) performing step (a) of the process as defined in the invention or any of the embodiments of this aspect,
(i.2) adding to the reaction medium the molecule of interest; and
(i.3) performing step (b) of the process of the invention;
or, alternatively, the process comprising:
(ii.1) incubating the self-standing catecholamine-based membrane with the molecule of interest after performing step b).

Step (i.2) is performed either directly adding to the reaction medium the molecule of interest as obtained from the supplier or in the form of an aqueous suspension (following manufacturer's instructions). The mild conditions used in the process of the invention advantageously allows the functionalization of the membrane in the own reaction medium wherein the membrane has been formed, which, advantageously, simplifies the process, reducing time and cost. As the membrane is formed on air/water or air/liquid interface, the adding step can be performed by pouring the solution in the reaction medium or, in case that the membrane is covering the whole surface of the reaction medium, by injecting the molecule's solution to the reaction medium through the membrane. The latter does not negatively affect to the integrity of the membrane, which is a further indicium of the robustness and improved mechanical properties of the membranes of the invention.

In the case of step (ii.1), the membrane has already been isolated from the interface. Therefore, the membrane may be incorporated to a medium comprising the solution with the molecule of interest (and the side in contact with the medium is functionalized) or, alternatively, the membrane is incorporated to a medium and a solution with the molecule is injected in the solution. The functionalization side can be selected previously, depositing the membrane with the desired side in contact with the liquid (aqueous) phase containing the molecule of interest. The membranes have functional groups exposed on the surface that can be used as anchoring points.

The functionalization of membrane's surface with the molecule of interest can occur by covalent bond, adsorption, or electrostatic interaction. In the case of a covalent bond, the functionalization can be performed through nucleophilic or electrophilic attack depending on the chemical nature of the molecule of interest. For example the presence of acyl chloride (e.g., stearoyl chloride) could act as electrophile in presence of amino-terminated groups. On the other hand, amino groups (e.g., hexadecamine) or thiol groups (e.g., 1,8-octanedithiol) could act as nucleophilic molecules being covalently bonded to the ring of catechol-derivatives. Besides, the presence of carboxylic acids in the backbone of the molecule of interest can be used for the formation of an amide bond (peptide-like bonding) with the terminal amino groups exposed on the surface of the membranes. Finally, the physical adsorption of molecules could be induced due to the entrapment of the molecule of interest in the backbone of the membranes. In one embodiment of the invention, the molecule of interest is covalently bound to the membrane surface of the second aspect of the invention.

In one embodiment, optionally in combination with any of the embodiments provided above or below, the catecholamine-based membrane of the invention further comprises one or more molecules of interest selected from the group consisting of therapeutic molecules, and detection labels (fluorescent active moieties, nanoparticles or antibodies). More particularly, the molecule of interest is covalently bound to the membrane, particularly by an amide bond.

The covalent bond can be achieved by any routine protocol, such as by generating an amide bound between the free amino groups of the membrane of the invention and the free carboxylic groups of the molecule of interest. The coupling reaction, in this case, can be performed using (1-ethyl-3-(3-dimethylamino) propyl carbodiimide, hydrochloride (EDC) and N-Hydroxysuccinimide (NHS). As a common procedure, the formed membranes can be introduced in Petri dishes of bigger diameter with a buffer within the range pointed out above. Then, the EDC is injected in the aqueous phase. After an appropriate period of time, such as 30 min, the active (bio)molecule (e.g., antibacterial moiety, florescent dye, growth factor, etc.) together with the NHS agent is injected in the aqueous phase. The reaction is stirred slowly, below 500 rpm, for a period of time enough to guarantee the covalent bond formation, such as 60 min. Then, the functionalized membranes can be extracted from the Petri dish and washed. The functionalization can be controlled and induced independently on both sides of the membranes, enhancing the chemical Janus behaviour of the membranes. Other catechol derivatives with different functional groups as terminal thiols, amines or acyl chloride can also be functionalized through the nucleophilic or electrophilic attack to the exposed functional groups on the membranes or the rings in the catechol-derivatives. The procedure would be similar as previously described. Some parameters such as the pH or the solvent can be routinely adjusted or selected for activating the reaction between the molecule of interest and the membrane.

In one embodiment, optionally in combination with any of the embodiments provided above or below, the treatment comprises administering one or more further anti-tumoral active agents.

In one embodiment, optionally in combination with any of the embodiments provided above or below, the one or more anti-tumoral active agents are selected from the group consisting of toxins, alkylating agents such as cyclophosphamide, ifosfamide, or temozolomide; platinum-based compounds such as cisplatin, carboplatin, or oxaliplatin; antimetabolites such as 5-fluorouracil (5-FU), capecitabine, methotrexate, pemetrexed or gemcitabine; topoisomerase inhibitors such as irinotecan or etoposide; antimicrotubule agents such as vncristine, vinblastine, paclitaxel, or docetaxel; anthracyclines such as doxorubicin; tyrosine kinase inhibitors (TKls) such as imatinib, sorafenib, or sunitinib; and monoclonal antibodies (mAbs) such as bevacizumab, trastuzumab, or rituximab; and combinations thereof. In one embodiment, optionally in combination with any of the embodiments provided above or below, the one or more anti-tumoral active agents are selected from the group consisting of doxorubicin, cisplatin, carboplatin, paclitaxel, docetaxel, 5-fluorouracil, capecitabine, gemcitabine, irinotecan, oxaliplatin, vincristine, vinblastine, etoposide, cyclophosphamide, ifosfamide, methotrexate, pemetrexed, temozolomide, imatinib, sorafenib, sunitinib, bevacizumab, trastuzumab, rituximab and combinations thereof.

In one embodiment, optionally in combination with any of the embodiments provided above or below, the treatment comprises further administering temozolomide. In another particular embodiment, the catechol derivative is catechin and the amine is hexamethylenediamine and the treatment comprises further administering temozolomide. As shown in the examples, a synergistic effect was obtained when CATH-M was administered with temozolomide. In a particular embodiment, the catechol derivative, the amine, and the active agent are present in amounts sufficient to produce a synergistic effect. Therefore, in an embodiment, catechin, the amine and temozolomide are are present in amounts sufficient to produce a synergistic effect are present in amounts sufficient to produce a synergistic effect.

The term "synergy or synergistic" is used herein refers to the fact that the effect observed by the combination of the membrane and the active agent is greater than the sum of the effects (additive effect) obtained independently with each one of the components of the combination.

In one embodiment, optionally in combination with any of the embodiments provided above or below, the treatment comprises irradiating before administering the anti-tumoral active agents.

In one embodiment, optionally in combination with any of the embodiments provided above or below, the self-standing catecholamine membrane and the one or more anti-tumoral active agents are administered simultaneously, sequentially, or separately.In one embodiment, optionally in combination with any of the embodiments provided above or below, the cancer is selected from the group consisting of breast cancer, colorectal cancer, lung cancer, brain cancer, ovarian cancer, head and neck cancer, pancreatic cancer, esophageal cancer, gastric cancer, liver cancer, prostate cancer, kidney cancer, bladder cancer, endometrial cancer, soft tissue sarcomas, bone cancer, testicular cancer, cervical cancer, thyroid cancer. In one embodiment, optionally in combination with any of the embodiments provided above or below, the cancer is brain cancer. In one embodiment, optionally in combination with any of the embodiments provided above or below, the brain cancer is glioblastoma.

In one embodiment, optionally in combination with any of the embodiments provided above or below, the treatment comprises placing the self-standing catechol-amine membrane in the surgical bed after a tumour resection. The self-standing catecholamine-based membrane according to the invention have demonstrated bioadhesion, biocompatibility, and the capacity to selectively eliminate cancer cells (particularly glioblastoma cells) and thus, they are adequate to be placed in the surgical bed after tumour resection.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### 1. MATERIALS

The catechol-based compounds (pyrocatechol, caffeic acid, dopamine, 4-methylcatechol, pyrogallol, and catechin) as well as the amine compounds of formula (II) (hexamethylenediamine, tris(2-aminoethyl)amine, dodecamethylenediamine, cystamine, pentaethylenehexamine, and norspermidine) were purchased from Sigma-Aldrich (Merck, Madrid, Spain) and used without further purification. Type 1 ultrapure water from a Milli-Q filtration system (Millipore, Burlington, MA, USA) was used in all experiments unless otherwise specified. In some experiments, different water-based buffers were used: phosphate-buffered saline (PBS, pH 7.4, Sigma-Aldrich) and aqueous carbonate buffer (pH 9.1) (dissolving 1.89 g of NaHCO₃ and 265 mg of Na₂CO₃ in 250 mL of Milli-Q water).

### 2. METHODS

### 2.1 Physicochemical and morphological characterization

Fourier transformed Infra-Red (FT-IR). Surface FT-IR experiments have been performed with the Hyperion 2000 FT-IR microscope (Bruker Optik GmbH, Ettlingen, Germany) in reflection mode equipped with a nitrogen-cooled mercury-m cadmium-telluride (MCT) detector (InfraRed Associates, Inc., Stuart, FL, USA) using a 15 × reflection objective, a gold mirror as a reference and scanning for 30 min with a resolution of 4 cm-1. The membranes were attached on a gold substrate. All the data was treated with OPUS version 7.5 (Bruker) and OriginPro (version 8.0988, OriginLab Corporation, Northampton, MA, USA) software.

X-ray photoelectron spectroscopy (XPS). XPS measurements were performed with a Phoibos 150 analyser (SPECS EAS10P GmbH, Berlin, Germany) in ultra-high vacuum conditions (based pressure 10-10 mbar, residual pressure around 10-7 mbar). Monochromatic Al Ka line was used as X-ray source (1486.6 eV and 300 W). The electron energy analyser was operated with pass energy of 50 eV. The hemispherical analyser was located perpendicular to the sample surface. The data was collected every eV with a dwell time of 0.5 s. A flood gun of electrons, with energy lower than 20 eV, was used to compensate the charge. The reference was set for C-C at 284.8 eV All the data was treated with CasaXPS version 2.3.17PR1.1 (Casa Software LTD, Teignmouth, UK) and OriginPro software.

Scanning Electron Microscopy (SEM). The morphology and thickness of the membranes was determined by scanning electron microscopy (SEM, FEI Quanta 650 FEG, Thermo Fisher Scientific, Eindhoven, The Netherlands) in secondary electron mode with a beam voltage of 10 kV. Samples were coated with a 5 nm layer of gold/palladium 60/40 layer (Emitech K550X Sputter Coater) before the observation. In the case of membranes and substrates containing cells, after fixation with 2% paraformaldehyde (PFA) in PBS for 1 h, samples were dehydrated through ethanol serial dilution series until 100 %. Afterwards, samples were dried under cabin airflow and coated with a 5 nm layer of gold/palladium.

Degradation test. The stability of the CATH-M and PYRO-M membranes was evaluated in water, ethanol 100%, pH 4 solution (HCl/sodium acetate), pH 10 solution (Na2CO3/NaHCO3), PBS, cell culture media (see next section) or trypsin (Gibco 25200072). Five ml of each solution were added to sterile 20 ml glass vials containing 2 discs of CATH-M or PYRO-M membranes. The degradation tests were performed at 37°C for 1, 2, 3, 4 and 8 months. After each time point, the membranes were removed, washed in water and further characterised by SEM.

### 2.2 Biological assays

Cell culture. Glioblastoma cell lines were routinely cultured and maintained in 100 mm cell culture dishes (Falcon^{™} 353003, Fisher Scientific), using 10 ml of Dulbecco's modified Eagle's medium (DMEM), supplemented with 100 µg/ml streptomycin, 100 U/ml penicillin, and 10 % heat-inactivated fetal bovine serum (FBS) (Invitrogen), from now on culture media. Cells were incubated at 37 °C in a saturating humidity atmosphere composed of 95% air and 5% CO2 (Fisherbrand^{™} CO2 Incubator Isotemp^{™}). After achieving 85-90% of confluence, cells were rinsed with PBS (100 mM, pH 7.4) and incubated at 37 °C for 3 min with 0.05% trypsin-EDTA (Trypsin) until dissociated. Afterwards, culture media was added to neutralize trypsin and the resulting suspension was centrifuged at 200 g for 5 min. Finally, the pellet was resuspended in culture media and, unless otherwise indicated, the cell density was adequately adjusted to conduct each experiment. The LN229 huma glioblastoma-derived cells (ATCC CRL-2611, 2010) were tested during entire study. Besides, four commercial cell lines (LN18 (ATCC CRL-2610, 2010)), A172 (ATCC CRL-1620, 2010), U87-MG (ATCC HTB-14, 2010) and U251-MG (ECACC 09063001, 2013)), two primary cultures (MSO4 and MSO7) and rat primary cortical astrocytes and neurons were used during specific tests, as representative tumoral and healthy nervous system cells. Moreover, A549 (ATCC CCL-185, 2015), PANC-1 (ATCC CRL-1469, 2015), CACO-2 (ATCC HTB-37, 2024), SAOS-2 (ATCC HTB-85, 2007) and HELA (ATCC CCL-2, 2012), were selected as representative tumoral cell lines for lungs, pancreas, colorectal, bone and cervical cancer, respectively. All procedures were performed in a biosafety level 2 laminar flow cabins in sterile conditions. The skilled person would know alternative cell lines and/or strains and culture conditions to be used for the intended purpose.

Isolation and culture of cortical astrocytes from rat. Primary cultures of cortical astrocytes were prepared from 1-day-old Sprague-Dawley rats following the protocol described previously. Cells were plated into 10 mm dishes containing a coverslip at a density of 0.3 × 106 viable cells/ml in DMEM supplemented with 10% fetal bovine serum (FBS), 50 units/ml penicillin and 50 µg/ml streptomycin. The cultures were incubated at 37 °C in a humidified atmosphere of 5% CO2/95% air and used at 21 days in vitro.

Cell viability. A double staining was used to evaluate the cytotoxic effect of the treatments performed in a 96-well plate (Falcon^{™} 353072, Fisher Scientific). After the desired incubation time of the cells and the membranes in 50 µl of culture media, 50 µl more of culture media with Propidium Iodide (PI, 1µg/ml) and Hoechst 33342 (H42, 2 µg/ml) were added to each well without removing the membranes (unless otherwise specified). Then, plates were incubated at room temperature protected from light for 30 min. H42 can pass through intact cell membranes, but PI can only enter cells that have suffered membrane damage. Finally, samples were examined under a Nikon ECLIPSE TE2000-E microscope, equipped with epifluorescence optics for visualizing fluorescent signals and a Hamamatsu ORCA-ER photographic camera for capturing high-quality images. Images were collected for each dye and bright field at various locations for each experimental condition. Cell death percentage was quantified as the percentage of cells with damaged membranes (PI-positive) relative to the total number of cells (H42-positive). Cell viability or death was expressed as the mean ± standard deviation for each condition, relative to the control (percentage), with a minimum of N=5, unless otherwise specified. Cell counting was performed using Fiji software.

Bacteria growth conditions. Escherichia coli (E. coli, MG 1655) and Methicillin-resistant Staphylococcus aureus (MRSA, CECT 9951) were selected as representative gram-negative and gram-positive bacteria, respectively. The selection of these strains was mainly due to their impact in current human health and relevance in research stated by the WHO and UN and their implication in brain surgery's infection. The skilled person would know alternative cell lines and/or strains and culture conditions to be used for the intended purpose. Initially, an aliquot from the bacteria was streaked on a 100 mm Petri dish containing Miller's Luria-Bertani (LB) with agar and incubated for 24 h at 37 °C (Forma^{™} Series II Water-Jacketed CO2 Incubator) under a saturating humidity atmosphere composed of 95 % air and 5 % CO2. For each experiment, a single colony was selected and grown in 10 ml of Miller's LB for 24 h at 37 °C. Bacteria cultures were resuspended in NaCl saline solution at 0.9 % and the optical density (OD600) of the suspension was adjusted (Fisherbrand^{™} Cell Density Meter Model 40) to 0.2 for E. coli, and 0.3 for MRSA. In every case, 100 mm Petri dish plates with Miller's LB with agar were used for bacteria growth and antibacterial tests. All procedures were performed under biosafety level 2 laminar flow cabins and sterile conditions.

Antibacterial properties in suspension. After obtaining the respective OD600 for each bacteria strain, 50 µl of suspension was added per well in a 96 well plates (Falcon^{™} 353072, Fisher Scientific), introducing inside of them a disc of 6 mm of CATH-M, PYRO-M, or nothing as control, followed by incubation at 37° for 24 h. Subsequently, the plate was sonicated for 1 min (Elmasonic S 30H) and serial dilutions were performed. Plates were seeded following the already described methodology for single plate-serial dilution spotting (SP-SDS) and kept incubating at 37°C for 24 h. Finally, viable colony-forming units (CFU) were counted afterwards, with a total number of repetitions of N = 12 for each condition. To determine ROS generation in base of bacteria/membrane selected, after the 24 h incubation, 50 µl of MAK143 kit was added to each well and incubated at room temperature for 30 min to finally read the fluorescence intensity (N=6) (Varioskan^{™} LUX, Thermo Scientific^{™} and λexcitation = 490 nm/λemission = 525 nm).

Cell Migration. To determine the influence of the membranes in the migration of the cells, 8 µm pore diameter inserts were used (Falcon^{®} 353097^{™}, Fisher Scientific). The corelation between migration, H2O2 production and survivability was assessed. First, for migration, a 24 well plate (Falcon^{®} 353047^{™}, Fisher Scientific) was filled with 800 µl of culture media, then no discs (control), 5 discs of 1, 2, 4 or 6 mm diameter of CATH-M, or 5 discs of 6 mm diameter of PYRO-M were added and remained incubating at 37° for 6 h. Subsequently, the insert was introduced in the well (containing or not membranes) and seeding 200 µl of culture media with 4 x 10⁴ cells over the inserts. After incubating for 16 h at 37°C, the cells that migrated to the lower face of the insert were quantified. To achieve that, cells were fixed with PFA at 4% final for 1 h, followed by the removal of the attached cells in the upper part of the insert (non-migrated cells) with a cotton swab. Afterwards, inserts were generously washed, stained with H42 in PBS and observed by fluorescence microscopy the remaining cells in the lower face of the insert (migrated cells). Three independent tests, with N=3 each one, were performed. The H2O2 production in the lower chamber was measured after the 16 h of incubation with the inserts and the membranes using the kit of OxiVision^{™} Green hydrogen peroxide sensor (see next section).

Cell Adhesion. In a 96 well plate, 100 µl/well of culture media were added, inserting a CATH-M or PYRO-M membrane, or not (control). Subsequently, 100 µl with 4 x 104 LN229 cells were seeded in each well. After 3 h incubation at 37°C, media was carefully aspirated and 200 µl of 1/5 of Trypsin in PBS were added and subsequently incubated for 30 min. Then, the plate was consistently hit to detach the affected cells, moving the supernatant to a new well (detached cells) and the membranes to another one (still attached cells). Besides, 100 µl of culture media with H42 (2 µg/ml final) were added to both cases to neutralize the Trypsin and to visualize the cells by fluorescence (N=4).

### 3. SYNTHESIS OF THE MEMBRANES OF THE INVENTION

The synthesis of the membranes was performed after combining the amines hexamethylenediamine (HMDA or -M), tris(2-aminoethyl)amine (TRI2), dodecamethylenediamine (DODE), cystamine (CYS), pentaethylenehexamine (PETH) or norspermidine (NORE) with different catechol derivatives: pyrocatechol (PYRO), caffeic acid (CAFF), pyrogallol (GALL), dopamine (DOPA), 4-methylcatechol (4MET) or catechin (CATH), according to the proportions indicated in the table 1 below:

**Table 1. Reference codes for the synthetized membranes according to the invention, catechol-amine molar ratio and catechol concentration.**

| **Reference** | **Catechol** | **Amine** | **molar ratio (catechol-amine)** | **[Catechol] (mM)** |
|---|---|---|---|---|
| 1P | PYRO | HMDA | 1 - 1.5 | 50 |
| 2P | PYRO | TRI2 | 1 - 2.8 | 100 |
| 3P | PYRO | DODE | 1 - 2 | 50 |
| 4P | PYRO | CYS | 1 - 2 | 200 |
| 5P | PYRO | PETH | 1 - 1 | 50 |
| 6P | PRYO | NORE | 1 - 1 | 50 |
| 1D | DOPA | HMDA | 1 - 2.5 | 50 |
| 2D | DOPA | TRI2 | 1 - 3 | 100 |
| 3D | DOPA | DODE | 1 - 2 | 50 |
| 4D | DOPA | CYS | 1 - 2 | 50 |
| 1M | 4MET | HMDA | 1 - 1.5 | 100 |
| 2M | 4MET | TRI2 | 1 - 2 | 100 |
| 3M | 4MET | DODE | 1 - 2 | 50 |
| 4M | 4MET | CYS | 1 - 2 | 200 |
| 1G | GALL | HMDA | 1 - 2 | 100 |
| 2G | GALL | TRI2 | 1 - 3 | 100 |
| 3G | GALL | DODE | 1 - 2 | 50 |
| 4G | GALL | CYS | 1 - 2 | 50 |
| 1CH | CATH | HMDA | 1 - 2.5 | 50 |
| 2CH | CATH | TRI2 | 1 - 3.4 | 50 |
| 1CF | CAFF | HMDA | 1 - 2 | 75 |
| 4CF | CAFF | CYS | 1 - 2 | 50 |

The selected catechol derivative and its corresponding amount of amine were weighted separately and dissolved in a final volume of 50 ml of water. Both reagents where mixed and poured in a sterile 15 cm diameter polypropylene Petri dish. In the case of CATH-M film, the CATH ligand was dissolved with 45 ml of sterile water, sonicated and heated. An autoclave-sterilised 15 cm glass dish was used for the membrane synthesis. The synthesis was performed for 48 h at 25 °C, obtaining a membrane in the interface between the air/water interface. Afterwards, the membranes were removed from their plates, washed three times with 15 ml of sterile water (until water was clean) and stored in 10 cm sterile Petri dish until their use. In every case, membranes were washed and cut to the final size inside 15 ml phosphate buffered saline (PBS) or saline solution (NaCl 0.9%) right before exposing them to cells or bacteria, respectively. All procedures were performed under biosafety level 2 laminar flow cabins to ensure sterile conditions.

### 4. RESULTS AND DISCUSSION

### 4.1. Physicochemical characterization

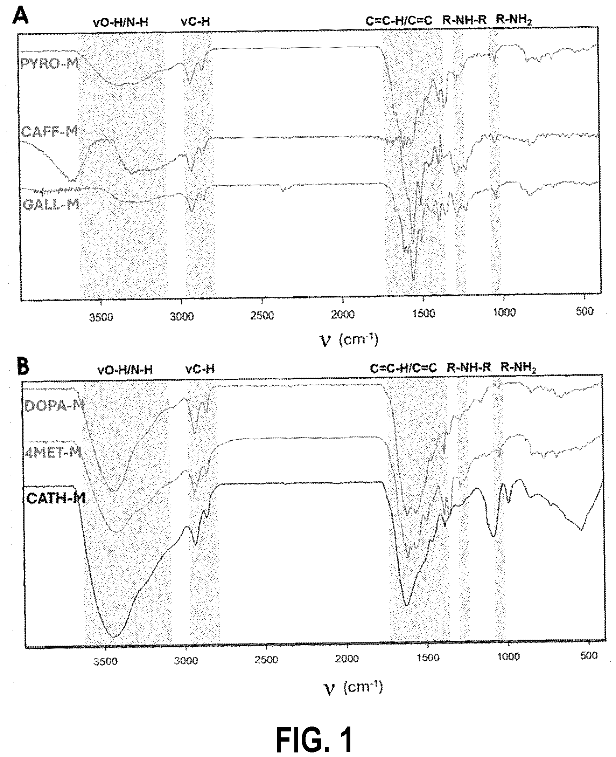

The polymerization of the membranes was thoroughly investigated using various techniques. Initially, FTIR analysis was conducted to identify the primary bonding structures. The results clearly indicated the presence of both amine and catechol/quinonic moieties in all membranes (FIG. 1). The spectra consistently exhibited similar features, including a characteristic broad band around 3250 cm⁻¹, attributed to the stretching vibrations of hydroxyl and amine (-NH₂) groups from phenol- and amine-based ligands, respectively. Additionally, the peak around 2850 cm⁻¹ was associated with aliphatic carbons (C-H), mainly present in the HMDA ligand. Notably, the peak at 1260 cm⁻¹ could be assigned to a secondary amine binding to an alkyl or aromatic ring, indicating successful catecholamine polymerisation. Finally, the peaks at 1700 cm⁻¹ and around 1500 cm⁻¹ were attributed to quinones (C=O) and C=C-H and C=C vibrations from the catecholic/quinonic rings, respectively. Furthermore, specific groups were identified. For instance, in DOPA-M, a peak at 1000 cm⁻¹ corresponded to free amine (-NH₂) groups, present in both HMDA and DOPA. On the other hand, the carboxylic groups (-COOH) present in CAFF-M were confirmed by a distinct band at 1270 cm⁻¹. Notably, in the case of CATH-M, the intensity of the peak corresponding to -OH groups was significantly higher compared to the other membranes (FIG. 1).

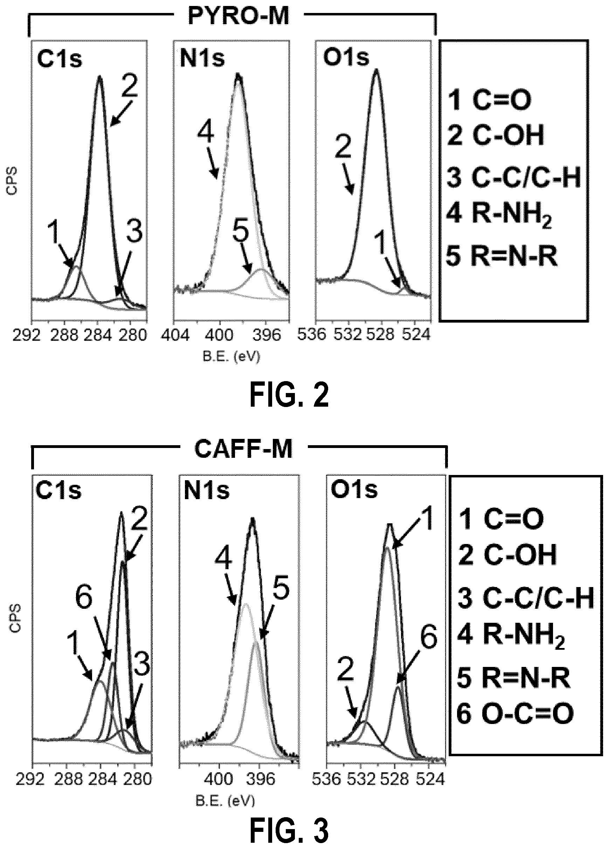

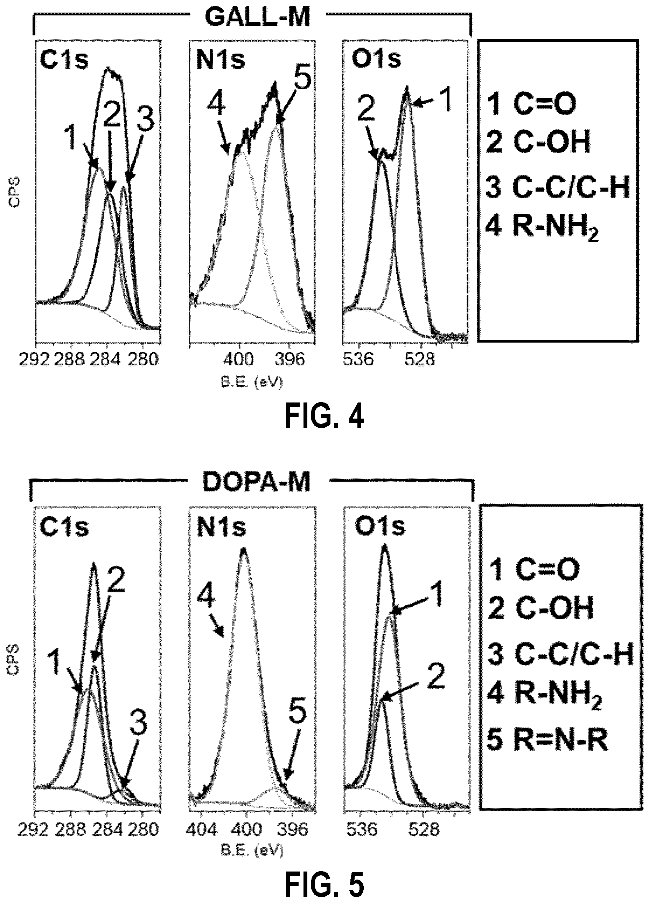

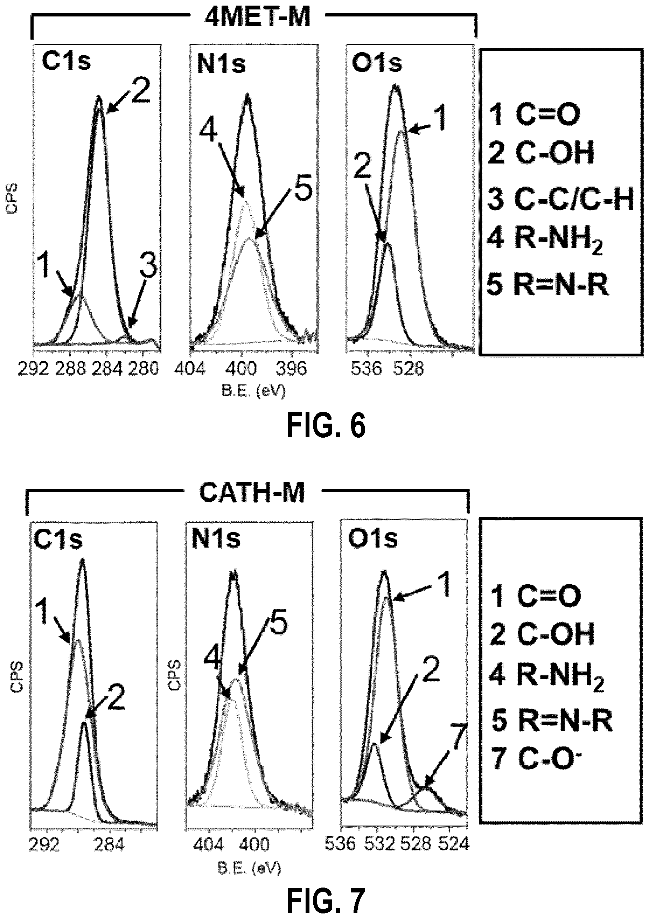

The most superficial composition was characterized to determine the exposed functional groups. This is relevant as it can be determinant to establish different interactions in the biointerface between the membrane, the tissue, and the cells. For this, high-resolution X-ray photoelectron spectroscopy (XPS) curve-fitting was used (FIG. 2 to FIG. 7). In all the synthesized membranes, C1s spectra showed three different chemical environments. Apart from the aliphatic component, the existence of the other two contributions confirmed that catechol coexists in its two forms: fully reduced (C-OH) and quinonic (C=O). In the case of CATH-M the C=O band was significantly higher. Moreover, O1s spectra of CAT-H includes a third one corresponding to the semiquinone form (C-O-). In the case of N1s spectra, it showed free (R-NH ₂) and aliphatic (R-N-R) amine-related contributions, associating the first one to unreacted amine tail ends of the HMDA and the second one to the result of cross-linking. The presence of hydroxyl and quinones groups is of special interest due to offering interaction and strong adhesion and furthermore the possibility of being further oxidized.

### 4.2. Morphological characterization

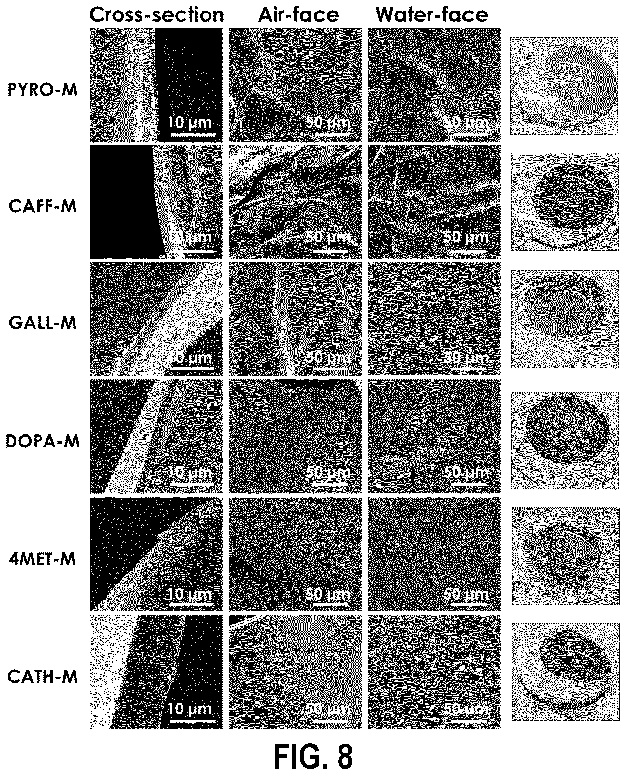

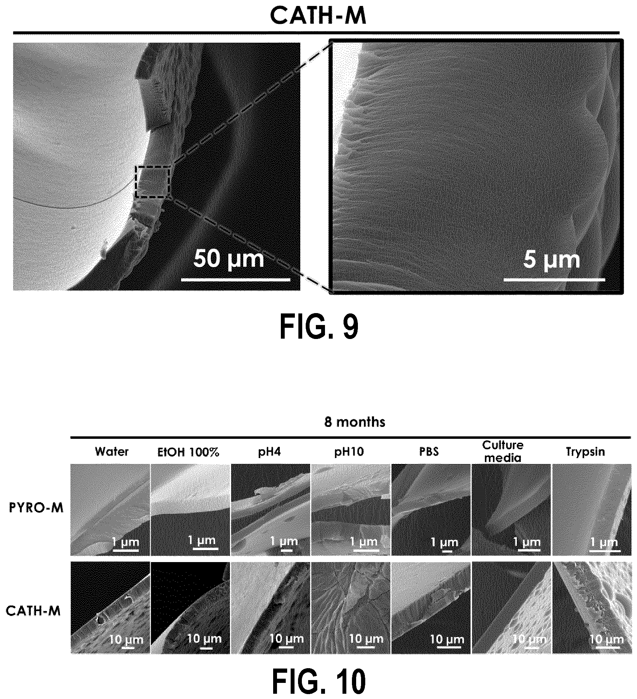

The choice of catechol derivative influenced not only the color and the chemical composition but the physical properties of the resulting membranes. For instance, PYRO was characterized for inducing the most homogeneous and flexible membranes. While GALL-based membranes have a considerable heterogenicity, seem to have an enhanced surface adhesion. The combination of HMDA with the catechol derivatives DOPA or CAFF, produced membranes with an easy manipulability and flexibility, respectively. In the case of 4MET, the resulting membranes was the most brittle, and CATH allowed for the formation of the thickest and most manipulable membrane. From a morphological point of view, scanning electron microscopy (SEM) showed that the six different membranes shared some patterns in their morphology and a Janus-like morphology (FIG. 8). While the air-face in all the cases was characterized by a flat or mostly flat surface (without major differences), the water-face yielded a particle-embedded face, observing in this case a higher variability between membranes (being PYRO-M the softest and the CATH-M the rougher). In terms of thickness, most of the membranes measured between 1 and 3 µm. PYRO-M was the thinnest, ranging from 0.7 to 1 µm, while CATH-M was significantly thicker, measuring between 10 and 12 µm (FIG. 8). Although the membranes were initially hydrophobic when completely dried, they were able to absorb water and become turgid, thereby turning hydrophilic. This suggests a permeable nature, despite the absence of a porous structure in the cross-section analysis, as shown in FIG. 9.

### 4.3. Degradation and stability

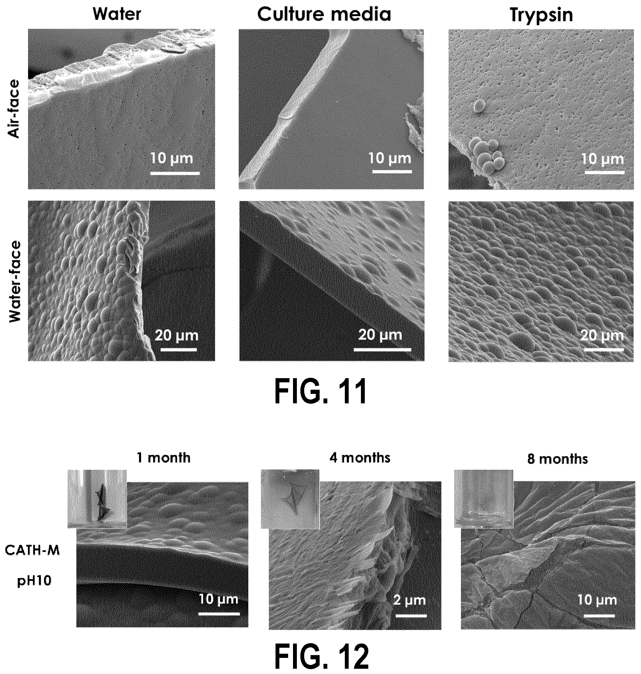

To evaluate the biodegradability of the membranes in different media, CATH-M and PYRO-M were stored for up to 8 months at 37°C in each of the following: water, 100% ethanol, pH 4 solution, pH 10 solution, phosphate-buffered saline (PBS), culture media, or trypsin. The evolution of the membranes was monitored over time and characterized using SEM (FIG. 10). While the PYRO-M showed only slight changes after 8 months, its macroscopic shape was altered when immersed in pH 10 solution. In contrast, the CATH-M displayed more noticeable changes under basic conditions, becoming more transparent within the first month and causing the medium to change color and ultimately degrading after 8 months under these conditions (FIG. 12). These observations suggest that the change in pH may induce changes in the bonding structure, leading to membrane weakening and highlighting its reactivity. Additionally, subtle differences were observed on the surface of the CATH-M membrane after 8 months when comparing water, culture media, and trypsin (FIG. 11). In cell culture media, the CATH-M surface became smoother, with nearly complete closure of the pores on the air-facing side and a more even appearance on the water-facing side. This was likely attributed to interactions with proteins and amino acids present in the culture media. Conversely, exposure to trypsin resulted in a slightly eroded surface with more rugosities and membrane protuberances, particularly on the air-face side. This effect is probably due to the protease activity of trypsin, which degrades it over time.

Although the best results for application in the human body were obtained for CATH-M, these findings highlight the potential of the membranes, as it demonstrates both stability and controlled biodegradability. The membranes remain stable during the necessary period for the expected treatment, ensuring their functionality and effectiveness. Over time, they progressively degrade, minimizing the risk of long-term complications.

### 4.4. Biological assays

### 4.4.1. Cytotoxicity activity screening

To comprehensively evaluate the cytotoxic activity against cancer cells, the all synthesized membranes were subjected to a screening assessment against a type of cancer, the human glioblastoma cell line LN229. A standardized experimental protocol was employed, involving the incubation of 2 × 104 LN229 cells with individual membrane discs for 24 h. Following this incubation period, the percentage of cell death was quantified using a combination of Hoechst 33342 (H42) and propidium iodide (PI) staining. H42 staining enabled the enumeration of total cells, while PI staining specifically targeted dead cells.

While all of the polyphenol-based membranes exhibited increased cytotoxicity in comparison to control (no membrane incubated with cells), the CATH-M membrane demonstrated a remarkable anti-tumoral effect. The CATH-M membrane induced cell death in approximately 90% of the LN229 cells, surpassing the cytotoxicity levels observed with the other five membranes.

Due to the results observed in LN229 cell line, the research was expanded to four additional established human glioblastoma-derived cell lines (LN18, A172, U87, and U251) and two primary cultures isolated from patients (MSO4 and MSO7). Initial seeding densities were set at 1.8 × 10⁴, 1.8 × 10⁴, 1.5 × 10⁴, 1 × 10⁴, 2.4 × 10⁴, and 2.6 × 10⁴ cells/well respectively. After a 24-hour incubation period, all six glioblastoma-derived cell lines exhibited a similar pattern of cell death as previously observed in LN229. Specifically, CATH-M consistently induced cell death rates around 80% in most of the cases, reaffirming its broad cytotoxic spectrum against glioblastoma cells. Furthermore, and in order to prove the broad applicability of the CATH-M membrane, the same experiment was performed with the tumoral cell lines A549, PANC-1, CACO-2 and SAOS-2 and HELA, which are derived from human lung carcinoma, pancreatic carcinoma, colorectal adenocarcinoma and osteosarcoma, respectively. In this case, 1 × 10⁴, 2 × 10⁴, 2 × 10⁴, 2.5 × 10⁴ and 1 × 10⁴ cells/well were seeded. After the 24 hours of incubation, the cell death induced by CATH-M (1CH) remained around the 80% in all the cases. Then, the same procedure was repeated for the rest of the membranes of Table 1. Briefly, the cytotoxicity of the membranes was tested against LN18, A172, U87, U251, MSO4, MSO7, A549, PANC-1, CACO-2, SAOS-2 and HELA, as described above for CATH-M, and after the 24 hours of incubation, the cell death induced by membranes with reference codes 1D, 2D, 1M, 2M, 1G, 2G, 3G, 4G and 2CH was higher than 60% in all cell lines tested. The rest of the membranes had toxicity ranging from 10 to 25% for all cell lines.

### 4.4.2. Antibacterial activity

The antibacterial properties were also evaluated. Methicillin-Resistant S. aureus (MRSA) and E. coli were selected as representative Gram-positive and Gram-negative bacteria, respectively. While PYRO-M exhibited a reduction of approximately 50% in Gram-negative E. coli, CATH-M demonstrated exceptional antibacterial activity after remaining inside a dense bacterial suspension for 24 h, achieving a Colony Forming Units (CFU) reduction of over 99.999% for MRSA and 99.99% for E. coli.

The importance of having intrinsic antimicrobial activity in a patch for potential glioblastoma post-surgery treatment cannot be overstated. The objective is to reduce the risk of post-operative infections, which are a major concern in neurosurgical procedures. By effectively eliminating pathogenic bacteria, these membranes can enhance patient outcomes, reduce the need for additional antibiotic treatments, and ultimately contribute to a more successful recovery process.

### 4.4.3. Ex vivo adhesion and brain conformability

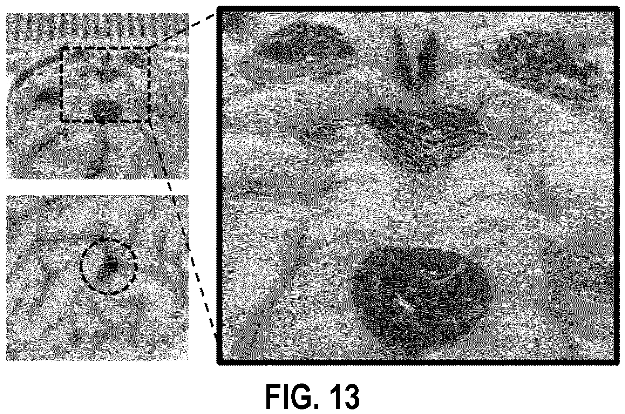

As a proof of concept, the self-standing membrane having the most promising anticancer properties, CATH-M, was selected for the assessment of the adhesion properties for its use in brain and glioblastoma post-surgical treatment, where adhesion in humid environments is required. Consequently, to ensure the adhesion properties of the CATH-M membrane, ex vivo assays were conducted. CATH-M was cut into round discs and placed as patches in various gyrus-sulcus areas of an ex vivo pig brain (FIG. 13). Notably, CATH-M demonstrated excellent conformability, adapting its shape to the brain surface regardless of position, and could be effortlessly introduced or removed from the cavity without breaking. Once positioned in the desired area, CATH-M remained securely in place, maintaining its adhesion even under high humidity conditions. This ensures that CATH-M can effectively remain in place and perform its intended function without displacement, vital for a successful post-surgical treatment.

### 4.4.4. Comparison with standard of care treatments

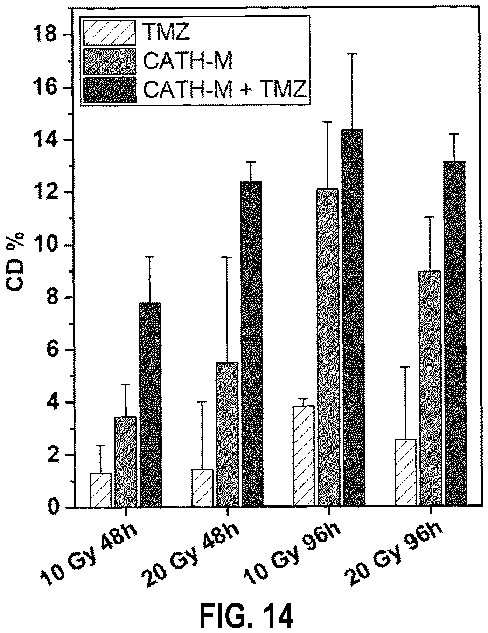

To investigate the potential interactions between CATH-M treatment and existing glioblastoma therapies, a comparative analysis of its cytotoxicity against TMZ was conducted. Their combined effects on LN229 cells (4.0 × 10⁴ cells/well) subjected to irradiation doses of 10 or 20 Gray (Gy), followed by 48 and 96 h incubation and then treated for 24 h with the membrane, was also assessed. It was revealed that CATH-M consistently exhibited superior cytotoxicity compared to TMZ (FIG. 14). Furthermore, the concurrent administration of CATH-M and TMZ (0.25 mM) yielded a higher effect across all exposure time. These results suggested that integrating CATH-M with current glioblastoma treatment protocols (irradiation plus TMZ) could potentially enhance synergistically therapeutic outcomes without compromising the existing standard of care.

### 4.4.5. Influence in cell adhesion and migration

To investigate the effects of CATH-M on LN229 cell behaviour, 4 × 10⁴ cells/well were seeded onto glass (control) or the membranes and incubated them for 24 h. Subsequent SEM analysis revealed that while LN229 cells exhibited normal morphology on control substrates, those seeded on CATH-M displayed significant morphological alterations, characterized by invaginations, retraction, and signs of membrane damage.

For comparison, the effect of CATH-M on non-tumoral healthy cells was analysed. For this, 2 × 10⁴ cells/well rat primary cortical astrocytes were cultured directly on the membranes. Interestingly, SEM observations revealed that these cells were largely unaffected by CATH-M, maintaining a morphology like the control. These results highlighted that CATH-M has a specific targeting cytotoxic effect for tumoral cells, while healthy cells are unaffected and can further grow and proliferate.

On the other hand, cell migration is one of the main mechanisms for tumor progression. To quantitatively assess the effect of CATH-M on cell migration, cell culture inserts with LN229 cells seeded in the upper chamber were used and varying amounts of CATH-M membranes introduced into the lower chamber as potential chemotactic stimulus. Interestingly, after 16 h of incubation, it was observed that by increasing the amount of CATH-M the migration decreased, while for lower concentrations enhanced the migration by up to 10% compared to the control, suggesting a chemoattractant effect. This chemoattractant effect could play an important role as cell trap.

CATH-M, particularly at higher concentrations, significantly increased cell death, with a near 90% mortality rate at the highest membrane concentration. SEM analysis revealed that the majority of LN229 cells exposed to high amounts of CATH-M exhibited a damaged spherical morphology and were predominantly located in the upper chamber of the insert, supporting the migration and viability results.

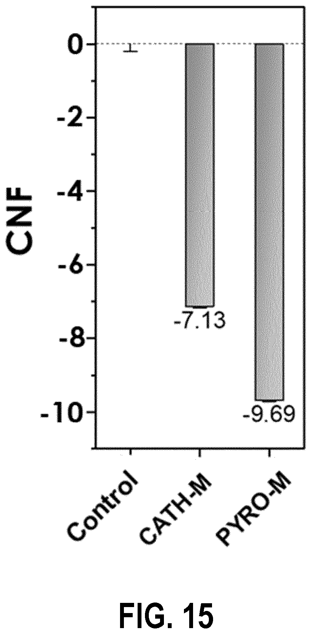

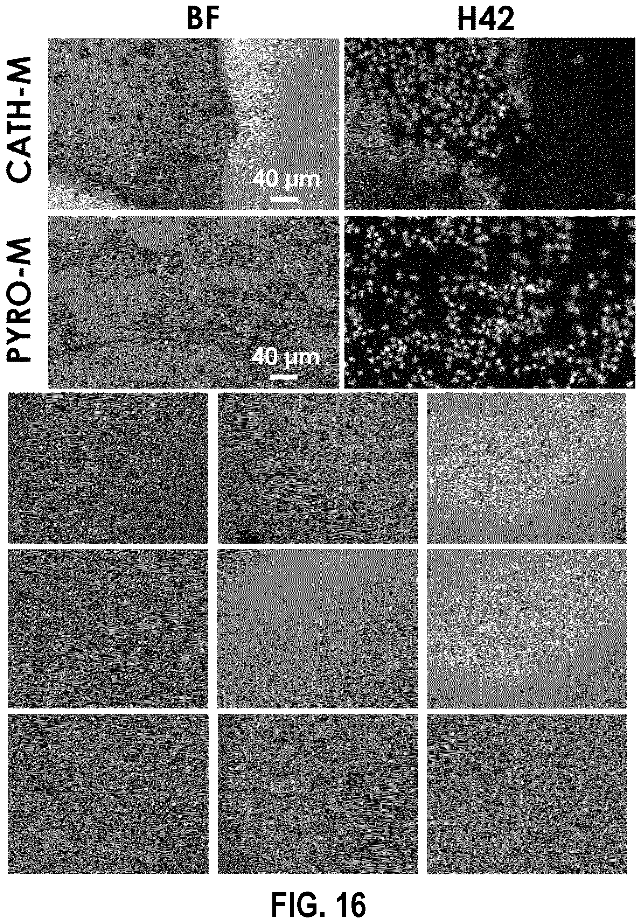

Overall, these observations suggest that while CATH-M can enhance LN229 cell migration under specific conditions, the observed decrease in migration at higher concentrations was likely due to increased cell death. Furthermore, the adhesion strength of LN229 cells to the bottom of a treated cell-culture plate (control) with both CATH-M and PYRO-M was compared. For this, cells were seeded and subsequently incubated for 3 h over the treated plastic (96-well plate bottom) or membranes. After trypsinisation for 30 min, the detached cells in each condition (FIG. 15 and FIG.16) were quantified. Notably, the number of cells detached from membranes were at least 7 times lower than from the control, even after protease treatment, demonstrating strong cell-membrane adhesion. These results highlighted that both CATH-M and PYRO-M membranes, had significantly enhanced cell adhesion compared to the control. This strong adhesion is likely due to the broad interaction range of polyphenols and their exposed functional groups with the surrounding environment, making them highly effective in promoting cell-membrane interactions.

## Claims

1. A self-standing catecholamine-based membrane for use in the treatment of cancer, wherein the self-standing catecholamine-based membrane is obtainable by a process comprising the steps of:
a) crosslinking a catechol derivative with an amine selected from the group consisting of:
a.1) a known aliphatic amine hydrocarbon of formula (II)
A-B-A' (II)
wherein
A and A' are the same or different and represent -NR₁R'₁,
B represents a (C₁-C₂₀)alkylene; (C₁-C₂₀)alkylene substituted with one or more substituents selected from the group consisting of: -OH, halogen, -NO₂, cyano, -O-(C₁-C₁₀)alkyl, -C(O)OR₂, and -NR₃R'₃; (C₂-C₂₀)alkenylene; (C₂-C₂₀)alkenylene substituted with one or more substituents selected from the group consisting of: -OH, halogen, -NO₂, cyano, -O-(C₁-C₁₀)alkyl, -C(O)OR₄, and -NR₅R'₅; (C₂-C₂₀)alkynylene; (C₂-C₂₀)alkynylene substituted with one or more substituents selected from the group consisting of: -OH, halogen, -NO₂, cyano, -O-(C₁-C₁₀)alkyl, -C(O)OR₆, and -NR₇R'₇; a known 3- to 20-membered heteroalkylene; a known 3- to 20-membered heteroalkylene substituted with one or more substituents selected from the group consisting of: - OH, halogen, -NO₂, cyano, -O-(C₁-C₁₀)alkyl, -C(O)OR₈, and NR₉R'₉; a known 3- to 20-membered heteroalkenylene; and a known 3- to 20-membered heteroalkenylene substituted with one or more substituents selected from the group consisting of: -OH, halogen, -NO₂, cyano, -O-(C₁-C₁₀)alkyl, -C(O)OR₁₀, and -NR₁₁R'₁₁;
A is bound to the first atom member forming part of the B biradical hydrocarbon backbone;
A' is bound to the last atom member forming part of the B biradical hydrocarbon backbone;
wherein the "first" and "last" atom members are identified reading the B biradical hydrocarbon backbone from left to right or vice versa;
R₁, R'₁, R₃, R'₃, R₅, R'₅, R₇, R'₇, R₉, R'₉, R₁₁, and R'₁₁ are the same or different and are selected from the group consisting of: -H; (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of -OH, -NO₂, cyano, -O-(C₁-C₁₀)alkyl, -C(O)OR₁₂, and -NR₁₃R'_{13;}
R₂, R₄, R₆, R₈, R₁₀ and R₁₂ are independently selected from the group consisting of - H; (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of -OH, -NO₂, cyano, -O-(C₁-C₁₀)alkyl, -C(O)OR₁₄and -NR₁₅R'_{15;}
R₁₃, R'₁₃, R₁₄, R₁₅ and R'₁₅ are the same or different and are selected from the group consisting of: -H; (C₁-C₁₀)alkyl; (C₁-C₁₀)haloalkyl; (C₂-C₁₀)alkenyl; and (C₂-C₁₀)alkynyl;
a "known 3- to 20-membered heteroalkylene" means a known saturated chain consisting of from 3 to 20 members selected from the group consisting of C(Rₓ)₂, CRₓ, -N-, -NR'ₓ-, -S-, and -O-, provided that: (a) at least one of the members is -N-, -NRₓ-, -S-, or -O-; and (b) the first and the last members forming the backbone of the heteroalkylene are carbon atoms;
a "known 3 to 20-membered heteroalkenylene" means a known unsaturated chain consisting of from 3 to 20 members selected from the group consisting of C(Rₓ)₂, -CRₓ-, -N-, -NR'ₓ-, -S-, and -O-, provided that: (a) at least one of the members is -N-, -NRₓ-, -S-, or -O-; (b) the first and the last members forming the backbone of the heteroalkylene are carbon atoms; and (c) includes one or more double bonds;
Rₓ is independently selected from the group consisting of -H; -OH; (C₁-C₁₀)alkyl; (C₂-C₁₀)alkenyl; (C₂-C₁₀)alkynyl; (C₁-C₁₀)haloalkyl; O-(C₁-C₁₀)alkyl; -O-(C₂-C₁₀)alkenyl; -O-(C₂-C₁₀)alkynyl; nitro, -NRₓ₁Rₓ₂; (C₁-C₁₀)alkyl substituted with one or more substituents selected from the group consisting of -OH, -NO₂, cyano, -O-(C₁-C₁₀)alkyl, -C(O)OR₁₆, and -NR₁₇R'₁₇; and halogen; and
R_{X1}, Rₓ₂, R₁₆, R₁₇, R'₁₇, and R'ₓ are independently selected from the group consisting of -H, (C₁-C₁₀)alkyl, (C₂-C₁₀)alkenyl, (C₂-C₁₀)alkynyl, and (C₁-C₁₀)haloalkyl; and
a.2) an aromatic amine of formula (IIbis): 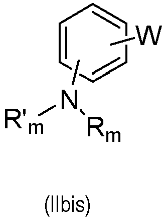 wherein W represents:
-NRₜR'ₜ or
*S-S-L; wherein (*) denotes that the S atom of W radical is bound to a carbon atom forming part of the aromatic ring; and
L is selected from the group consisting of: (C₁-C₁₀)alkyl substituted with one or more substituents selected from the group consisting of -OH, -NO₂, cyano, -O-(C₁-C₁₀)alkyl, -C(O)OR₂₈, and -NR₂₈R'₂₈, provided that at least one of the substituents is -NR₂₈R'₂₈; (C₁-C₁₀)haloalkyl substituted with one or more substituents selected from the group consisting of -OH, -NO₂, cyano, -O-(C₁-C₁₀)alkyl, -C(O)OR₂₈, and -NR₂₈R'₂₈, provided that at least one of the substituents is -NR₂₈R'₂₈; (C₂-C₁₀)alkenyl substituted with one or more substituents selected from the group consisting of -OH, -NO₂, cyano, -O-(C₁-C₁₀)alkyl, -C(O)OR₂₈, and -NR₂₈R'₂₈, provided that at least one of the substituents is -NR₂₈R'₂₈; (C₂-C₁₀)alkynyl substituted with one or more substituents selected from the group consisting of -OH, -NO₂, cyano, -O-(C₁-C₁₀)alkyl, -C(O)OR₂₈, and -NR₂₈R'₂₈, provided that at least one of the substituents is -NR₂₈R'₂₈; and a known aromatic ring having 5 or 6 members selected from the group consisting of -CR_{y}-, -N-, -O-, -NR'ᵥ, -S-;
Rₘ, R'ₘ, Rₜ and R'ₜ are the same or different and are selected from the group consisting of: H, (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of -OH, -NO₂, cyano, -O-(C₁-C₁₀)alkyl, -C(O)OR₂₉, and -NR₃₀R'₃₀; and a known aromatic ring having 5 or 6 members selected from the group consisting of -CR_{z}-, -N-, -NR'_{z,} -O-, and -S-;
at least one of Rᵥ, is -NR₃₁R'₃₁ and the other(s) Rᵥ are selected from the group consisting of H, -NR₃₁R'₃₁, (C₁-C₁₀)alkyl; (C₁-C₁₀)haloalkyl; (C₂-C₁₀)alkenyl; (C₂-C₁₀)alkynyl; and (C₁-C₁₀)alkyl substituted with one or more substituents selected from the group consisting of -OH, -NO₂, cyano, -O-(C₁-C₁₀)alkyl, - C(O)OR₃₂, and -NR₃₃R'₃₃;
R_{z}, and R'_{z} are independently selected from the group consisting of: H, (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of -OH, -NO₂, cyano, -O-(C₁-C₁₀)alkyl, -C(O)OR₃₄, and -NR₃₅R'₃₅; and
R₂₈, R'₂₈, R₂₉, R₃₀, R'₃₀, R₃₁, R'₃₁, R₃₂, R₃₃, R'₃₃, R₃₄, R₃₅, and R'₃₅ are independently selected from the group consisting of: -H; (C₁-C₁₀)alkyl; (C₁-C₁₀)haloalkyl; (C₂-C₁₀)alkenyl; and (C₂-C₁₀)alkynyl;
to create a catecholamine membrane in the air/liquid interface in the absence of any support, the crosslinking reaction being performed in a liquid medium, wherein both the catechol and the amine are soluble, at a pH comprised from 6.5 to 10, and under appropriate agitation; and b) isolating the membrane resulting from step (a) from the air/liquid interface.

2. The self-standing catecholamine-based membrane for use according to claim 1, wherein the amine of step a) is one of formula (II).

3. The self-standing catecholamine-based membrane for use according to claim 2, wherein A and A' are the same and represent -NHR'₁, particularly -NH₂.

4. The self-standing catecholamine-based membrane for use according to any one of the claims 2-3, wherein B represents (C₁-C₁₅)alkylene; (C₁-C₁₅)alkylene substituted as defined in claim 1; or a known 3- to 15-membered heteroalkylene as defined in claim 1.

5. The self-standing catecholamine-based membrane for use according to claim 1, wherein the amine is selected from the group consisting of: hexamethylenediamine, octamethylenediamine, dodecamethylenediamine, cystamine, tris-(3-aminopropyl)amine, tris-(2-aminopropyl)amine, and 4,4',4"-triaminotriphenylamine.

6. The self-standing catecholamine-based membrane for use according to any one of the previous claims, wherein the catechol derivative is one of formula (III): 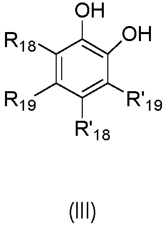 wherein
R₁₈, R₁₉, R'₁₈ and R'₁₉ are the same or different and are selected from the group consisting of:
-H;
-OH;
-NR₂₀R'₂₀;
halogen
(C₁-C₁₀)alkyl;
(C₂-C₁₀)alkenyl;
(C₁-C₁₀)alkyl substituted with one or more substituents selected from the group consisting of: -OH, halogen, nitro, cyano, (C₁-C₁₀)alkyl, (C₁-C₁₀)haloalkyl, -NR₂₁R'₂₁, -C(O)OR₂₂, and -O-(C₁-C₁₀)alkyl;
(C₂-C₁₀)alkenyl substituted with one or more substituents selected from the group consisting of: -OH, halogen, nitro, cyano, (C₁-C₁₀)alkyl, (C₁-C₁₀)haloalkyl, -NR₂₃R'₂₃, -C(O)OR₂₄, and -O-(C₁-C₁₀)alkyl;
a known ring system consisting of one or two rings, each one of the rings: (a) consisting of 5 or 6 members selected from the group consisting of -C(R_{y})₂-, -CR_{y}-, -N-, -NR'_{y}, -S-, and -O-; (b) being saturated, partially unsaturated or aromatic, and (c) being isolated, partially isolated or fused;
each one of the R_{y} is independently selected from the group consisting of -H, -OH, (C₁-C₁₀)alkyl, (C₂-C₁₀)alkenyl, (C₂-C₁₀)alkynyl, (C₁-C₁₀)haloalkyl, -O-(C₁-C₁₀)alkyl, nitro, -NR₂₅R₂₅, and halogen;
R'_{y} is selected from the group consisting of -H, (C₁-C₁₀)alkyl, (C₂-C₁₀)alkenyl, (C₂-C₁₀)alkynyl, and (C₁-C₁₀)haloalkyl;
R₂₀, R'₂₀, R₂₁, R'₂₁, R₂₃, R'₂₃, R₂₅, and R'₂₅ are the same or different and are selected from the group consisting of: -H; (C₁-C₁₀)alkyl; (C₁-C₁₀)haloalkyl; (C₂-C₁₀)alkenyl; (C₂-C₁₀)alkynyl; (C₁-C₁₀)alkyl substituted with one or more substituents selected from the group consisting of: -OH, halogen, nitro, cyano, (C₁-C₁₀)alkyl, (C₁-C₁₀)haloalkyl, -NR₂₆R'₂₆, -C(O)OR₂₇, and -O-(C₁-C₁₀)alkyl;
R₂₂ and R₂₄ are independently selected from the group consisting of: H, (C₁-C₁₀)alkyl, (C₁-C₁₀)haloalkyl, (C₂-C₁₀)alkenyl; and (C₂-C₁₀)alkynyl; and
R₂₆, R'₂₆ and R₂₇ are the same or different and are selected from the group consisting of: -H; (C₁-C₁₀)alkyl; (C₁-C₁₀)haloalkyl; (C₂-C₁₀)alkenyl; and (C₂-C₁₀)alkynyl.

7. The self-standing catecholamine-based membrane for use according to claim 6, wherein the catechol of formula (III) is one of formula (Illa), (IIIb), any stereoisomer thereof, or mixtures thereof: 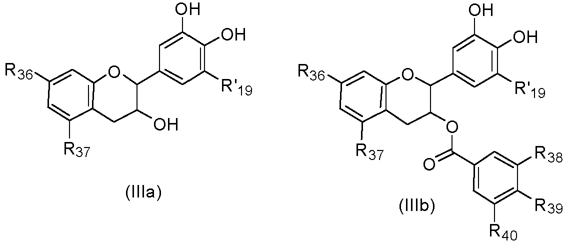 wherein
R'₁₉, R₃₆, R₃₇, R₃₈, R₃₉ and R₄₀ are the same or different and are selected from the group consisting of: -H; and -OH.

8. The self-standing catecholamine-based membrane for use according to any one of claims 1-6, wherein the catechol derivative is selected from the group consisting of pyrocatechol, dopamine, pyrogallol, caffeic acid and 4-methylcatechol.

9. The self-standing catecholamine-based membrane for use according to claim 1 or claim 8, wherein the catechol derivative is catechin and the amine is hexamethylenediamine.

10. The self-standing catecholamine-based membrane for use according to any one of the previous claims, wherein the self-standing catecholamine-based membrane further comprises one or more molecules of interest selected from the group consisting of therapeutic molecules, cells, growth factors, detection labels, and combinations thereof.

11. The self-standing catecholamine-based membrane for use according to any one of the previous claims, wherein the treatment further comprises administering one or more anti-tumoral active agents.

12. The self-standing catecholamine-based membrane for use according to claim 11, wherein the one or more anti-tumoral active agents are selected from the group consisting of toxins, alkylating agents, platinum-based compounds, anti metabolites, topoisomerase inhibitors, antimicrotubule agents, anthracyclines, tyrosine kinase inhibitors, monoclonal antibodies, , and combinations thereof.

13. The self-standing catecholamine-based membrane according to any one of the previous claims, wherein the cancer is selected from the group consisting of breast cancer, colorectal cancer, lung cancer, brain cancer, ovarian cancer, head and neck cancer, pancreatic cancer, esophageal cancer, gastric cancer, liver cancer, prostate cancer, kidney cancer, bladder cancer, endometrial cancer, soft tissue sarcomas, bone cancer, testicular cancer, cervical cancer, thyroid cancer.

14. The self-standing catecholamine-based membrane for use according to claim 13, wherein the cancer is brain cancer, particularly glioblastoma.

15. The self-standing catecholamine-based membrane for use according to any one of the previous claims, wherein the treatment comprises placing the self-standing catechol-amine membrane in the surgical bed after a tumour resection.
